(19) Europäisches Patentamt
European Patent Office
Office européen des brevets

(11) Publication number: **0 622 355 A2**

# (12) EUROPEAN PATENT APPLICATION

(21) Application number: **94106783.7**

(22) Date of filing: **29.04.94**

(51) Int. Cl.[5]: **C07C 251/76**, C07C 251/80, C07C 327/58, C07C 255/66, C07D 295/04, C07D 207/335, C07D 333/22, C07D 307/52, C07D 213/53, C07D 213/02, C07D 265/30

(30) Priority: **30.04.93 JP 128401/93**

(43) Date of publication of application: **02.11.94 Bulletin 94/44**

(84) Designated Contracting States: **CH DE ES FR GB IT LI**

(71) Applicant: **NIHON NOHYAKU CO., LTD.**
**1-2-5, Nihonbashi**
**Chuo-ku Tokyo (JP)**

(72) Inventor: **Nishida, Tateki**
**18-17, Minoyama Miyuki**
**Yawata-shi (JP)**
Inventor: **Tajima, Sohkichi**
**10-9-301, Nagai-2-chome,**
**Sumiyoshi-ku**
**Osaka-shi (JP)**
Inventor: **Kanno, Hideo**
**2-2-708, Shirakawa-3-chome**
**Ibaraki-shi (JP)**

(74) Representative: **Patentanwälte Grünecker, Kinkeldey, Stockmair & Partner**
**Maximilianstrasse 58**
**D-80538 München (DE)**

(54) **Benzylhydrazone derivates, a process for production thereof, and agricultural and horticultural fungicides.**

(57) A benzylhydrazone derivative represented by the general formula (I):

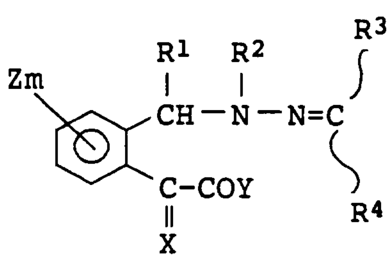

(I)

[wherein X is CH~$OR^5$ or N~$OR^5$ (wherein $R^5$ is a $C_{1-6}$alkyl group), Y is a $C_{1-6}$alkoxy group, a $C_{1-6}$alkylthio group, an unsubstituted amino group, or a mono- or di-substituted amino group having as the substituent(s) one or two $C_{1-6}$alkyl groups, each of Z's is a halogen atom, a $C_{1-6}$alkyl group or a halo-$C_{1-6}$alkyl groups, m is zero or an integer of 1 to 4, $R^1$ is a hydrogen atom, a $C_{1-6}$alkyl group or a halo-$C_{1-6}$alkyl group, each of $R^2$, $R^3$ and $R^4$ is a specific group] which is very effective against rice blast, cucumber downy mildew, barley powdery mildew, etc. at a low dosage; a process for producing said derivative; and an agricultural and horticultural fungicide containing said derivative as an active ingredient.

EP 0 622 355 A2

## BACKGROUND OF THE INVENTION

### Field of the invention

The present invention relates to benzylhydrazone derivatives represented by the following general formula (I), a process for producing the same, and agricultural and horticultural fungicides:

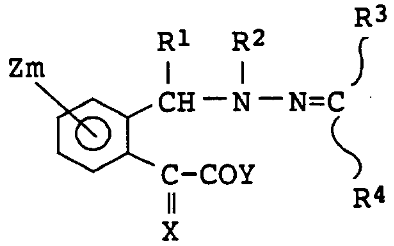

(I)

wherein X is a group represented by the formula:

CH~$OR^5$

(wherein $R^5$ is a $C_{1-6}$alkyl group) or a group represented by the formula:

N~$OR^5$

(wherein $R^5$ is as defined above), Y is a $C_{1-6}$alkoxy group, a $C_{1-6}$alkylthio group, an unsubstituted amino group, or a mono- or di-substituted amino group having as the substituent(s) one or two $C_{1-6}$alkyl groups which may be the same or different, Z's, which may be the same or different, are halogen atoms, $C_{1-6}$alkyl groups or halo-$C_{1-6}$alkyl groups, m is zero or an integer of 1 to 4, $R^1$ is a hydrogen atom, a $C_{1-6}$alkyl group or a halo-$C_{1-6}$alkyl group, $R^2$ is a formyl group; a $C_{1-6}$alkylcarbonyl group; a halo-$C_{1-6}$alkylcarbonyl group; a $C_{1-6}$alkylsulfonyl group; a halo-$C_{1-6}$alkylsulfonyl group; an unsubstituted aminocarbonyl group; a mono- or disubstituted aminocarbonyl group having one or two substituents which may be the same or different and are selected from the group consisting of formyl group and $C_{1-6}$alkyl groups; a $C_{1-6}$alkoxycarbonyl group; a $C_{1-6}$alkoxycarbonyl-$C_{1-6}$alkylcarbonyl group; a cyano-$C_{1-6}$alkylcarbonyl group; a $C_{1-6}$alkylthiocarbonyl group; an unsubstituted phenylcarbonyl group; a substituted phenylcarbonyl group having 1 to 5 substituents which may be the same or different and are selected from the group consisting of halogen atoms, $C_{1-6}$alkyl groups, halo-$C_{1-6}$alkyl groups, $C_{1-6}$alkoxy groups and halo-$C_{1-6}$alkoxy groups; an unsubstituted phenyl-$C_{1-6}$alkylcarbonyl group; a substituted phenyl-$C_{1-6}$alkylcarbonyl group having on the phenyl ring 1 to 5 substituents which may be the same or different and are selected from the group consisting of halogen atoms, $C_{1-6}$alkyl groups, halo-$C_{1-6}$alkyl groups, $C_{1-6}$alkoxy groups and halo-$C_{1-6}$alkoxy groups; an unsubstituted phenoxycarbonyl group; a substituted phenoxycarbonyl group having on the phenyl ring 1 to 5 substituents which may be the same or different and are selected from the group consisting of halogen atoms, $C_{1-6}$alkyl groups, halo-$C_{1-6}$alkyl groups, $C_{1-6}$alkoxy groups and halo-$C_{1-6}$alkoxy groups; an unsubstituted benzyloxycarbonyl group; a substituted benzyloxycarbonyl group having on the phenyl ring 1 to 5 substituents which may be the same or different and are selected from the group consisting of halogen atoms, $C_{1-6}$alkyl groups, halo-$C_{1-6}$alkyl groups, $C_{1-6}$alkoxy groups and halo-$C_{1-6}$alkoxy groups; or a di-substituted phosphonyl group having as the substituents two $C_{1-6}$alkyl groups which may be the same or different, and $R^3$ and $R^4$, which may be the same or different, are hydrogen atoms; cyano groups; $C_{1-6}$alkyl groups; halo-$C_{1-6}$alkyl groups; $C_{3-6}$cycloalkyl groups; cyano-$C_{1-6}$alkyl groups; $C_{2-6}$alkenyl groups; $C_{3-6}$cycloalkenyl groups; $C_{1-6}$alkoxy groups; halo-$C_{1-6}$alkoxy groups; $C_{1-6}$alkylthio groups; halo-$C_{1-6}$alkylthio groups; $C_{1-6}$alkylsulfinyl groups; $C_{1-6}$alkylsulfonyl groups; $C_{2-6}$alkenylthio groups; $C_{2-6}$alkynylthio groups; $C_{1-6}$alkoxy-$C_{1-6}$alkyl groups; $C_{1-6}$alkoxy-$C_{1-6}$alkylthio groups; $C_{1-6}$alkylthio-$C_{1-6}$alkyl groups; $C_{1-6}$alkylsulfonyl-$C_{1-6}$alkyl groups; $C_{3-6}$cycloalkylthio groups; $C_{1-6}$alkoxycarbonyl-$C_{1-6}$alkylthio-$C_{1-6}$alkyl groups; $C_{1-6}$alkoxycarbonylthio groups; unsubstituted phenyl groups; substituted phenyl groups having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkylsulfinyl groups, $C_{1-6}$alkylsulfonyl groups, unsubstituted aminocarbonyl group, mono- or di-substituted aminocarbonyl groups having one or two substituents which may be the same or different and are selected from the group consisting of $C_{1-6}$alkyl groups and $C_{1-6}$alkylcarbonyl groups, cyano-$C_{1-6}$alkoxy groups, $C_{1-6}$alkoxycarbonyloxy groups, unsubstituted amino group, mono- or di-substituted amino groups having one or two substituents which may be the same or different and are selected from the group consisting of formyl group, $C_{1-6}$alkyl groups and $C_{1-6}$alkylcarbonyl groups, $C_{1-6}$alkoxycarbonyl groups, unsubstituted aminocarbonyloxy group, mono- or disubstituted aminocarbonyloxy groups having one or two substituents which may be the same or different and are selected from $C_{1-6}$alkyl groups, methylenedioxy group, unsubstituted phenyl group, substituted phenyl groups having 1 to 5 substituents which may be the same or different and are selected from the group consisting of halogen atoms, $C_{1-6}$alkyl groups and $C_{1-6}$alkoxy groups, unsubstituted phenoxy group, substituted phenoxy groups having 1 to 5 substituents which may be the same or different and are selected from the group consisting of halogen atoms, cyano group, nitro group, $C_{1-6}$alkyl groups and halo-$C_{1-6}$alkyl groups, unsubstituted phenyl-$C_{1-6}$alkyl groups, substituted phenyl-$C_{1-6}$alkyl groups having on the phenyl ring 1 to 5 substituents which may be the same or different and are selected from the group consisting of halogen atoms, cyano group, nitro group, $C_{1-6}$alkyl groups and halo-$C_{1-6}$alkyl groups, unsubstituted phenyl-$C_{1-6}$alkoxy groups, substituted phenyl-$C_{1-6}$alkoxy groups having on the phenyl ring 1 to 5 substituents which may be the same or different and are selected from the group consisting of halogen atoms, cyano group, nitro group, $C_{1-6}$alkyl groups and halo-$C_{1-6}$alkyl groups, unsubstituted morpholino group, substituted morpholino groups having as the substituent(s) one or more $C_{1-6}$alkyl groups which may be the same or different, unsubstituted pyrimidinyloxy group, substituted pyrimidinyloxy groups having as the substituent(s) one or more $C_{1-6}$alkyl groups which may be the same or different, unsubstituted morpholinocarbonyl group, substituted morpholinocarbonyl groups having as the substituent(s) one or more $C_{1-6}$alkyl groups which may be the same or different, and $C_{3-4}$alkylene groups which each form a bicyclic ring together with the adjacent carbon atoms of the phenyl ring; unsubstituted phenyl-$C_{1-6}$alkylthio groups; substituted phenyl-$C_{1-6}$alkylthio groups having on the phenyl ring 1 to 5 substituents which may be the same or different and are selected from the group consisting of $C_{1-6}$alkyl groups and halo-$C_{1-6}$alkyl groups; unsubstituted phenylsulfonyl groups; substituted phenylsulfonyl groups having on the phenyl ring 1 to 5 substituents which may be the same or different and are selected from the group consisting of $C_{1-6}$alkyl groups and halo-$C_{1-6}$alkyl groups; unsubstituted phenylsulfonyl-$C_{1-6}$alkyl groups; substituted phenylsulfonyl-$C_{1-6}$alkyl groups having on the phenyl ring 1 to 5 substituents which may be the same or different and are selected from the group consisting of $C_{1-6}$alkyl groups and halo-$C_{1-6}$alkyl groups; unsubstituted phenylthio-$C_{1-6}$alkyl groups; substituted phenylthio-$C_{1-6}$alkyl groups having on the phenyl ring 1 to 5 substituents which may be the same or different and are selected from the group consisting of $C_{1-6}$alkyl groups and halo-$C_{1-6}$alkyl groups; unsubstituted phenyl-$C_{1-6}$alkylthio-$C_{1-6}$alkyl groups; substituted phenyl-$C_{1-6}$alkylthio-$C_{1-6}$alkyl groups having on the phenyl ring 1 to 5 substituents which may be the same or different and are selected from the group consisting of $C_{1-6}$alkyl groups and halo-$C_{1-6}$alkyl groups; unsubstituted amino-$C_{1-6}$alkyl groups; mono- or di-substituted amino-$C_{1-6}$alkyl groups having one or two substituents which may be the same or different and are selected from the group consisting of $C_{1-6}$alkyl groups, halo-$C_{1-6}$alkyl groups, $C_{1-6}$alkylcarbonyl groups, unsubstituted phenylcarbonyl group, substituted phenylcarbonyl groups having on the phenyl ring 1 to 5 substituents which may be the same or different and are selected from the group consisting of $C_{1-6}$alkyl groups and halo-$C_{1-6}$alkyl groups, phenyl group and benzyl group; furfurylthio groups; furfurylthio-$C_{1-6}$alkyl groups; unsubstituted morpholino-$C_{1-6}$alkyl groups; substituted morpholino-$C_{1-6}$alkyl groups having as the substituent(s) one or more $C_{1-6}$alkyl groups which may be the same or different; groups represented by the formula:

$$-CO{\sim}R^6$$

(wherein $R^6$ is a $C_{1-6}$alkyl group; a halo-$C_{1-6}$alkyl group; a $C_{1-6}$alkoxy group; a halo-$C_{1-6}$alkoxy group; a $C_{1-6}$alkylthio group; a halo-$C_{1-6}$alkylthio group; an unsubstituted amino group; a mono- or di-substituted amino group having one or two substituents which may be the same or different and are selected from the group consisting of $C_{1-6}$alkyl groups, halo-$C_{1-6}$alkyl groups and phenyl group; an unsubstituted phenyl group; a substituted phenyl group having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, unsubstituted amino group, mono- or di-substituted amino groups having one or two substituents which may be the same or different and are selected from the group consisting of $C_{1-6}$alkyl groups and $C_{1-6}$alkylcarbonyl groups, unsubstituted phenyl group, substituted phenyl groups having 1 to 5 substituents which may be the same or different and are

selected from the group consisting of halogen atoms, $C_{1-6}$alkyl groups and halo-$C_{1-6}$alkyl groups, unsubstituted phenoxy group, substituted phenoxy groups having 1 to 5 substituents which may be the same or different and are selected from the group consisting of halogen atoms, cyano group, nitro group, $C_{1-6}$alkyl groups and halo-$C_{1-6}$alkyl groups, benzyl group, unsubstituted benzyloxy group, substituted benzyloxy groups having on the phenyl ring 1 to 5 substituents which may be the same or different and are selected from the group consisting of halogen atoms, $C_{1-6}$alkyl groups and halo-$C_{1-6}$alkyl groups, unsubstituted pyridyloxy group, substituted pyridyloxy groups having 1 to 4 substituents which may be the same or different and are selected from the group consisting of halogen atoms, $C_{1-6}$alkyl groups and halo-$C_{1-6}$alkyl groups, methylenedioxy group, and $C_{3-4}$alkylene groups which each form a bicyclic ring together with the adjacent carbon atoms of the phenyl ring; an unsubstituted benzyloxy group; a substituted benzyloxy group having on the phenyl ring 1 to 5 substituents which may be the same or different and are selected from the group consisting of halogen atoms, $C_{1-6}$alkyl groups, halo-$C_{1-6}$alkyl groups, $C_{1-6}$alkoxy groups, halo-$C_{1-6}$alkoxy groups, $C_{1-6}$alkylthio groups and halo-$C_{1-6}$alkylthio groups; or a 5- or 6-membered heterocyclic ring having 1 to 3 heteroatoms which may be the same or different and are selected from the group consisting of oxygen atom, sulfur atom and nitrogen atom, said heterocyclic ring being able to have one or more substituents selected from the group consisting of $C_{1-6}$alkyl groups, halo-$C_{1-6}$alkyl groups, $C_{1-6}$alkoxy groups and halo-$C_{1-6}$alkoxy groups); unsubstituted amino groups; mono- or di-substituted amino groups having one or two substituents which may be the same or different and are selected from the group consisting of $C_{1-6}$alkyl groups, halo-$C_{1-6}$alkyl groups, $C_{2-6}$alkenyl groups, $C_{2-6}$alkynyl groups, $C_{1-6}$alkoxy-$C_{1-6}$alkyl groups, di-$C_{1-6}$alkoxy-$C_{1-6}$alkyl groups, amino-$C_{1-6}$alkyl groups, substituted mono- or di-$C_{1-6}$alkylamino-$C_{1-6}$alkyl groups having as the substituent(s) one or more $C_{1-6}$alkyl groups which may be the same or different, unsubstituted phenyl group, substituted phenyl groups having 1 to 5 substituents which may be the same or different and are selected from the group consisting of halogen atoms, $C_{1-6}$alkyl groups, $C_{1-6}$alkoxy groups, $C_{1-6}$alkylthio groups and halo-$C_{1-6}$alkyl groups, pyridyl-$C_{1-6}$alkyl groups, and morpholino-$C_{1-6}$alkyl groups; unsubstituted phenyl-$C_{1-6}$alkyl groups; substituted phenyl-$C_{1-6}$alkyl groups having on the phenyl ring 1 to 5 substituents which may be the same or different and are selected from the group consisting of halogen atoms, $C_{1-6}$alkyl groups, halo-$C_{1-6}$alkyl groups, $C_{1-6}$alkoxy groups, halo-$C_{1-6}$alkoxy groups, $C_{1-6}$alkylthio groups and halo-$C_{1-6}$alkylthio groups; unsubstituted phenyl-$C_{2-6}$alkenyl groups; substituted phenyl-$C_{2-6}$alkenyl groups having on the phenyl ring 1 to 5 substituents which may be the same or different and are selected from the group consisting of halogen atoms, $C_{1-6}$alkyl groups and halo-$C_{1-6}$alkyl groups; naphthyl groups; 5- to 7-membered heterocyclic rings having 1 to 3 heteroatoms which may be the same or different and are selected from the group consisting of oxygen atom, sulfur atom and nitrogen atom; or 5- to 7-membered heterocyclic ring fused with a benzene ring or a $C_{4-6}$cycloalkane ring; the above heterocyclic rings being able to have one or more substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxycarbonyl groups, unsubstituted phenyl group, substituted phenyl groups having 1 to 5 substituents which may be the same or different and are selected from halogen atoms, benzyl group, pyridyl group, pyrimidyl group and dioxolane group, $R^3$ and $R^4$ being able to be taken together to represent a $C_{1-6}$-alkylene group or a $C_{2-6}$-alkenylene group, which may contain between adjacent carbon atoms of the carbon chain an oxygen atom, a sulfur atom, a nitrogen atom, a carbonyl group, or a nitrogen atom having a $C_{1-6}$alkyl or phenyl group bonded thereto, said alkylene or alkenylene group being able to have on the carbon chain one or more substituents which may be the same or different and are selected from the group consisting of $C_{1-6}$alkyl groups, halo-$C_{1-6}$alkyl groups, $C_{1-6}$alkoxy groups and halo-$C_{1-6}$alkoxy groups, and to form fused rings by condensation with a benzene ring.

Related Art

Japanese Patent Unexamined Publication Nos. 4-288045, 4-261147, 4-182461, 3-169842, 3-120246 and W093/15046, etc. disclose compounds such as acrylic esters or amides and describe them as useful as agricultural and horticultural fungicides.

SUMMARY OF THE INVENTION

The present inventors earnestly investigated for providing a more useful agricultural and horticultural fungicide and consequently found that the benzylhydrazone derivatives of the general formula (I) are novel compounds not known in any literature and have an excellent fungicidal effect at a low dosage, whereby the

present invention has been accomplished.

DETAILED DESCRIPTION OF PREFERRED EMBODIMENTS

In the definition of the substituents of the benzylhydrazone derivative of the general formula (I) of the present invention, the halogen atoms include chlorine atom, fluorine atom, bromine atom, iodine atom, etc. In the substituents, the term "$C_{1-6}$alkyl", for example, means an alkyl group having 1 to 6 carbon atoms, such as methyl, ethyl, n-propyl, i-propyl, n-butyl, i-butyl, s-butyl, t-butyl, n-pentyl or the like. The term "$C_{1-6}$alkoxy" means an alkoxy group having 1 to 6 carbon atoms, such as methoxy, ethoxy, n-propoxy, i-propoxy, n-butoxy, i-butoxy, s-butoxy, t-butoxy, n-pentyloxy, n-hexyloxy or the like. The term "$C_{1-6}$alkylthio" means an alkylthio group having 1 to 6 carbon atoms, such as methylthio, ethylthio, n-propylthio, i-propylthio, n-butylthio, i-butylthio, s-butylthio, t-butylthio, n-pentylthio, n-hexylthio or the like. The prefix "halo" is used for expressing that a group has as its substituent(s) one or more halogen atoms which may be the same or different. For example, the term "$C_{1-6}$haloalkyl group" means a substituted alkyl group of 1-6 carbon atoms having as the substituent(s) one or more halogen atoms which may be the same or different.

The benzylhydrazone derivative of the general formula (I) of the present invention can be produced, for example, by the following process.

Production process 1.

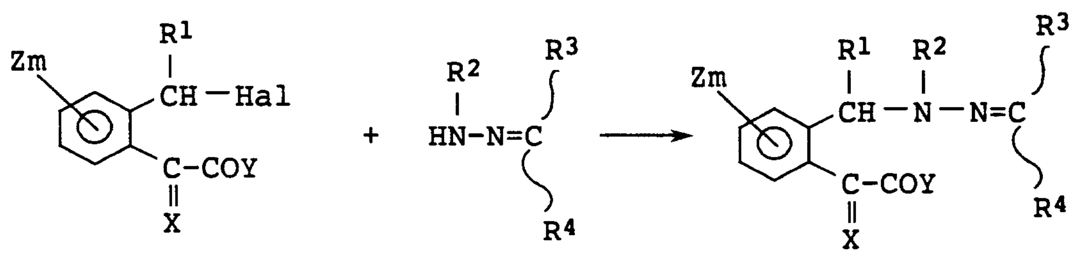

(II) (III) (I)

wherein $R^1$, $R^2$, $R^3$, $R^4$, X, Y, Z and m are as defined above, and Hal is a halogen atom.

The benzylhydrazone derivative of the general formula (I) can be produced by reacting a compound of the general formula (II) with a compound of the general formula (III) in the presence of a base or a silver compound, and an inert solvent.

As the inert solvent usable in this reaction, any inert solvent may be used so long as it does not markedly inhibit the progress of the reaction. There may be used, for example, alcohols such as methanol, ethanol, isopropanol, tert-butanol, diethylene glycol, etc.; ketones such as acetone, methyl ethyl ketone, cyclohexanone, etc.; ethers such as diethyl ether, diisopropyl ether, tetrahydrofuran, dioxane, monoglyme, diglyme, etc.; halogenated hydrocarbons such as dichloroethane, chloroform, carbon tetrachloride, tetrachloroethane, etc.; aromatic hydrocarbons such as benzene, chlorobenzene, nitrobenzene, toluene, etc.; nitriles such as acetonitrile, etc.; dimethylformamide; dimethyl sulfoxide; and water. These inert solvents may be used singly or as a mixture thereof.

Two-phase reaction can be carried out as the above reaction by using water and a water-insoluble inert solvent. In this case, there can be used phase transfer catalysts such as triethylbenzylammonium chloride, trioctylmethylammonium chloride, etc.

As the base used in the reaction, an inorganic base or an organic base may be used. As the inorganic base, there may be used, for example, carbonates or hydroxides of alkali metal atoms or alkaline earth metal atoms, such as sodium carbonate, potassium carbonate, calcium carbonate, sodium hydrogencarbonate, sodium hydroxide, potassium hydroxide, calcium hydroxide, etc., and hydrides of alkali metal atoms, such as lithium hydride, sodium hydride, etc. As the organic base, there may be used, for example, alkoxides of alkali metal atoms, such as sodium methoxide, potassium tertbutoxide, etc., diethylamine, triethylamine, pyridine, and benzyltrimethylammonium hydroxide. These bases may be used singly or as a

mixture thereof. The amount of the base used may be properly chosen in the range of 1 mole to excess moles per mole of the compound of the general formula (II).

As the silver compound usable in the reaction, silver oxide, for example, may be used. The amount of the silver compound used may be properly chosen in the range of 1 mole to excess moles per mole of the compound of the general formula (II).

Since the reaction is an equimolar reaction, it is sufficient that the compound of the general formula (II) and the compound of the general formula (III) are used in equimolar amounts, though either of them may be used in excess.

The reaction temperature is chosen in the range of -70 °C to the boiling point of the inert solvent used. It is preferably -40 °C to room temperature.

Although the reaction time is varied depending on the reaction temperature, the degree of reaction, etc., it is usually chosen in the range of 30 minutes to 48 hours.

After completion of the reaction, the desired compound is isolated from the reaction mixture by a conventional method, and if necessary, purified by column chromatography, recrystallization, etc., whereby the benzylhydrazone derivative of the general formula (I) can be produced.

Production process 2.

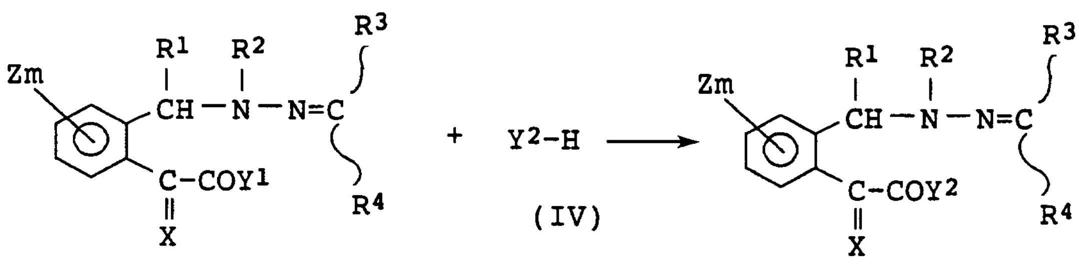

(I')

(I")

wherein $R^1$, $R^2$, $R^3$, $R^4$, $Y^1$ is a $C_{1-6}$alkoxy group or a $C_{1-6}$alkylthio group and $Y^2$ is an unsubstituted amino group or a mono- or di-substituted amino group having as the substituent(s) one or two $C_{1-6}$alkyl groups which may be the same or different, X, Z and m are as defined above.

A benzylhydrazone derivative of the general formula (I") can be produced by reacting a benzylhydrazone derivative of the general formula (I') with an amine of the general formula (IV) in the presence of an inert solvent.

As the inert solvent usable in this reaction, any inert solvent may be used so long as it does not markedly inhibit the progress of the reaction. There may be used, for example, alcohols such as methanol, ethanol, isopropanol, tert-butanol, diethylene glycol, etc.; halogenated hydrocarbons such as dichloromethane, chloroform, carbon tetrachloride, etc.; aromatic hydrocarbons such as benzene, toluene, xylene, etc.; ethers such as Methyl Cellosolve, diethyl ether, diglyme, dioxane, tetrahydrofuran, etc.; dimethylformamide; dimethyl sulfoxide; and water. These inert solvents may be used singly or as a mixture thereof.

Since the reaction is an equimolar reaction, it is sufficient that the benzylhydrazone derivative of the general formula (I') and the amine of the general formula (IV) are used in equimolar amounts, though the amine of the general formula (IV) is preferably used in excess.

The reaction temperature is chosen in the range of -70 °C to the boiling point of the inert solvent used. It is preferably -20 °C to room temperature.

Although the reaction time is varied depending on the reaction temperature, the degree of reaction, etc., it is usually chosen in the range of 30 minutes to 48 hours.

After completion of the reaction, the desired compound is isolated from the reaction mixture by a conventional method, and if necessary, purified by column chromatography, recrystallization, etc., whereby the benzylhydrazone derivative of the general formula (I") can be produced.

Typical compounds as the benzylhydrazone derivative of the general formula (I) are listed below, but they are not intended in any way to limit the scope of the present invention.

General formula (1)

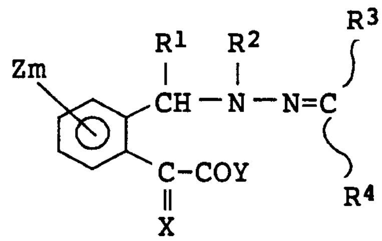

Table 1 ($R^1$=H, Ph=a phenyl group)

| No. | $R^2$ | $R^3$ | $R^4$ | X | Y | Zm |
|---|---|---|---|---|---|---|
| 1 | HCO- | Ph | $CH_3$ | N-$OCH_3$ | $OCH_3$ | H |
| 2 | $CH_3CO$- | $SCH_3$ | $SCH_3$ | N-$OCH_3$ | $OCH_3$ | H |
| 3 | $CH_3CO$- | $CH_2OCH_3$ | $CH_3$ | CH-$OCH_3$ | $OCH_3$ | H |
| 4 | $CH_3CO$- | $OCH_3$ | $CH_3$ | N-$OCH_3$ | $OCH_3$ | H |
| 5 | $CH_3CO$- | $SCH_3$ | $SCH_3$ | N-$OCH_3$ | $NHCH_3$ | H |
| 6 | $CH_3CO$- | $SCH_2CH=CH_2$ | $SCH_2CH=CH_2$ | N-$OCH_3$ | $OCH_3$ | H |
| 7 | $CH_3CO$- | $SCH_2CH=CH_2$ | $SCH_2CH=CH_2$ | N-$OCH_3$ | $NHCH_3$ | H |
| 8 | $CH_3CO$- | $SC_2H_4OC_2H_5$ | $SC_2H_4OC_2H_5$ | N-$OCH_3$ | $OCH_3$ | H |
| 9 | $CH_3CO$- | $SC_2H_4OC_2H_5$ | $SC_2H_4OC_2H_5$ | N-$OCH_3$ | $NHCH_3$ | H |
| 10 | $CH_3CO$- | Ph | $CH_3$ | N-$OCH_3$ | $OCH_3$ | H |
| 11 | $CH_3CO$- | Ph | $CH_3$ | N-$OCH_3$ | NHCH3 | H |
| 12 | $CH_3CO$- | Ph | $CH_3$ | CH-$OCH_3$ | $OCH_3$ | H |
| 13 | $CH_3CO$- | Ph | $C_2H_5$ | CH-$OCH_3$ | $OCH_3$ | H |
| 14 | $CH_3CO$- | Ph | i-$C_3H_7$ | N-$OCH_3$ | $OCH_3$ | H |
| 15 | $CH_3CO$- | Ph | i-$C_3H_7$ | N-$OCH_3$ | $NHCH_3$ | H |
| 16 | $CH_3CO$- | 4-Cl-Ph | H | N-$OCH_3$ | $OCH_3$ | H |
| 17 | $CH_3CO$- | 4-Cl-Ph | H | N-$OCH_3$ | NHCH3 | H |

- Cont'd -

Table 1 (Cont'd)

| No. | $R^2$ | $R^3$ | $R^4$ | X | Y | Zm |
|---|---|---|---|---|---|---|
| 18 | $CH_3CO-$ | 4-Cl-Ph | H | $CH-OCH_3$ | $OCH_3$ | H |
| 19 | $CH_3CO-$ | 2-Cl-Ph | $CH_3$ | $CH-OCH_3$ | $OCH_3$ | H |
| 20 | $CH_3CO-$ | 2-Cl-Ph | $CH_3$ | $N-OCH_3$ | $OCH_3$ | H |
| 21 | $CH_3CO-$ | 2-Cl-Ph | $CH_3$ | $N-OCH_3$ | $NHCH_3$ | H |
| 22 | $CH_3CO-$ | 3-Cl-Ph | $CH_3$ | $CH-OCH_3$ | $OCH_3$ | H |
| 23 | $CH_3CO-$ | 3-Cl-Ph | $CH_3$ | $N-OCH_3$ | $OCH_3$ | H |
| 24 | $CH_3CO-$ | 3-Cl-Ph | $CH_3$ | $N-OCH_3$ | $NHCH_3$ | H |
| 25 | $CH_3CO-$ | 4-Cl-Ph | $CH_3$ | $CH-OCH_3$ | $OCH_3$ | H |
| 26 | $CH_3CO-$ | 4-Cl-Ph | $CH_3$ | $N-OCH_3$ | $OCH_3$ | H |
| 27 | $CH_3CO-$ | 4-Cl-Ph | $CH_3$ | $N-OCH_3$ | $NHCH_3$ | H |
| 28 | $CH_3CO-$ | 4-Cl-Ph | $C_2H_5$ | $N-OCH_3$ | $OCH_3$ | H |
| 29 | $CH_3CO-$ | 4-Cl-Ph | $C_2H_5$ | $N-OCH_3$ | $NHCH_3$ | H |
| 30 | $CH_3CO-$ | 4-Cl-Ph | $C_2H_5$ | $CH-OCH_3$ | $OCH_3$ | H |
| 31 | $CH_3CO-$ | 2,4-$Cl_2$-Ph | $CH_3$ | $N-OCH_3$ | $OCH_3$ | H |
| 32 | $CH_3CO-$ | 2,4-$Cl_2$-Ph | $CH_3$ | $N-OCH_3$ | $NHCH_3$ | H |
| 33 | $CH_3CO-$ | 3,4-$Cl_2$-Ph | $CH_3$ | $N-OCH_3$ | $OCH_3$ | H |
| 34 | $CH_3CO-$ | 3,4-$Cl_2$-Ph | $CH_3$ | $N-OCH_3$ | $NHCH_3$ | H |
| 35 | $CH_3CO-$ | 2,5-$Cl_2$-Ph | $CH_3$ | $N-OCH_3$ | $OCH_3$ | H |
| 36 | $CH_3CO-$ | 2,5-$Cl_2$-Ph | $CH_3$ | $N-OCH_3$ | $NHCH_3$ | H |
| 37 | $CH_3CO-$ | 2,3,4-$Cl_2$-Ph | $CH_3$ | $N-OCH_3$ | $OCH_3$ | H |
| 38 | $CH_3CO-$ | 2,3,4-$Cl_2$-Ph | $CH_3$ | $N-OCH_3$ | $NHCH_3$ | H |
| 39 | $CH_3CO-$ | 3-Br-Ph | $CH_3$ | $N-OCH_3$ | $OCH_3$ | H |
| 40 | $CH_3CO-$ | 3-Br-Ph | $CH_3$ | $N-OCH_3$ | $NHCH_3$ | H |

- Cont'd -

Table 1 (Cont'd)

| No. | $R^2$ | $R^3$ | $R^4$ | X | Y | Zm |
|---|---|---|---|---|---|---|
| 41 | $CH_3CO$- | 4-Br-Ph | $CH_3$ | CH-$OCH_3$ | $OCH_3$ | H |
| 42 | $CH_3CO$- | 4-Br-Ph | $CH_3$ | N-$OCH_3$ | $OCH_3$ | H |
| 43 | $CH_3CO$- | 4-Br-Ph | $CH_3$ | N-$OCH_3$ | $NHCH_3$ | H |
| 44 | $CH_3CO$- | 3-F-Ph | $CH_3$ | N-$OCH_3$ | $OCH_3$ | H |
| 45 | $CH_3CO$- | 3-F-Ph | $CH_3$ | N-$OCH_3$ | $NHCH_3$ | H |
| 46 | $CH_3CO$- | 4-F-Ph | $CH_3$ | CH-$OCH_3$ | $OCH_3$ | H |
| 47 | $CH_3CO$- | 4-F-Ph | $CH_3$ | N-$OCH_3$ | $OCH_3$ | H |
| 48 | $CH_3CO$- | 4-F-Ph | $CH_3$ | N-$OCH_3$ | $NHCH_3$ | H |
| 49 | $CH_3CO$- | 4-F-Ph | $C_2H_5$ | N-$OCH_3$ | $NHCH_3$ | H |
| 50 | $CH_3CO$- | 2,4-$F_2$-Ph | $CH_3$ | N-$OCH_3$ | $OCH_3$ | H |
| 51 | $CH_3CO$- | 2,4-$F_2$-Ph | $CH_3$ | N-$OCH_3$ | $NHCH_3$ | H |
| 52 | $CH_3CO$- | 3,4-$F_2$-Ph | $CH_3$ | N-$OCH_3$ | $OCH_3$ | H |
| 53 | $CH_3CO$- | 3,4-$F_2$-Ph | $CH_3$ | N-$OCH_3$ | $NHCH_3$ | H |
| 54 | $CH_3CO$- | 2,5-$F_2$-Ph | $CH_3$ | N-$OCH_3$ | $OCH_3$ | H |
| 55 | $CH_3CO$- | 2,5-$F_2$-Ph | $CH_3$ | N-$OCH_3$ | $NHCH_3$ | H |
| 56 | $CH_3CO$- | 2,3-$F_2$-Ph | $CH_3$ | N-$OCH_3$ | $OCH_3$ | H |
| 57 | $CH_3CO$- | 2,3-$F_2$-Ph | $CH_3$ | N-$OCH_3$ | $NHCH_3$ | H |
| 58 | $CH_3CO$- | 3,5-$F_2$-Ph | $CH_3$ | N-$OCH_3$ | $OCH_3$ | H |
| 59 | $CH_3CO$- | 3,5-$F_2$-Ph | $CH_3$ | N-$OCH_3$ | $NHCH_3$ | H |
| 60 | $CH_3CO$- | 2,6-$F_2$-Ph | $CH_3$ | N-$OCH_3$ | $OCH_3$ | H |
| 61 | $CH_3CO$- | 2,6-$F_2$-Ph | $CH_3$ | N-$OCH_3$ | $NHCH_3$ | H |
| 62 | $CH_3CO$- | 2,4,5-$F_3$-Ph | $CH_3$ | N-$OCH_3$ | $OCH_3$ | H |
| 63 | $CH_3CO$- | 2,4,5-$F_3$-Ph | $CH_3$ | N-$OCH_3$ | $NHCH_3$ | H |

- Cont'd -

Table 1 (Cont'd)

| No. | $R^2$ | $R^3$ | $R^4$ | X | Y | Zm |
|---|---|---|---|---|---|---|
| 64 | $CH_3CO-$ | 2,3,4,5,6-$F_5$-Ph | $CH_3$ | N-$OCH_3$ | $OCH_3$ | H |
| 65 | $CH_3CO-$ | 2,3,4,5,6-$F_5$-Ph | $CH_3$ | N-$OCH_3$ | $NHCH_3$ | H |
| 66 | $CH_3CO-$ | 3-$NO_2$-4-Cl-Ph | $CH_3$ | N-$OCH_3$ | $OCH_3$ | H |
| 67 | $CH_3CO-$ | 3-$NO_2$-4-Cl-Ph | $CH_3$ | N-$OCH_3$ | $NHCH_3$ | H |
| 68 | $CH_3CO-$ | 3-$NO_2$-Ph | $CH_3$ | CH-$OCH_3$ | $OCH_3$ | H |
| 69 | $CH_3CO-$ | 4-$NO_2$-Ph | $CH_3$ | CH-$OCH_3$ | $OCH_3$ | H |
| 70 | $CH_3CO-$ | 4-CN-Ph | $CH_3$ | CH-$OCH_3$ | $OCH_3$ | H |
| 71 | $CH_3CO-$ | 4-CN-Ph | $CH_3$ | N-$OCH_3$ | $OCH_3$ | H |
| 72 | $CH_3CO-$ | 4-CN-Ph | $CH_3$ | N-$OCH_3$ | $NHCH_3$ | H |
| 73 | $CH_3CO-$ | 4-CN-Ph | $CH_3$ | CH-$OCH_3$ | $OCH_3$ | 5-Cl |
| 74 | $CH_3CO-$ | 2-$CH_3$-Ph | $CH_3$ | CH-$OCH_3$ | $OCH_3$ | H |
| 75 | $CH_3CO-$ | 2-$CH_3$-Ph | $CH_3$ | N-$OCH_3$ | $OCH_3$ | H |
| 76 | $CH_3CO-$ | 2-$CH_3$-Ph | $CH_3$ | N-$OCH_3$ | $NHCH_3$ | H |
| 77 | $CH_3CO-$ | 3-$CH_3$-Ph | $CH_3$ | N-$OCH_3$ | $OCH_3$ | H |
| 78 | $CH_3CO-$ | 3-$CH_3$-Ph | $CH_3$ | N-$OCH_3$ | $NHCH_3$ | H |
| 79 | $CH_3CO-$ | 4-$CH_3$-Ph | $CH_3$ | CH-$OCH_3$ | $OCH_3$ | H |
| 80 | $CH_3CO-$ | 4-$CH_3$-Ph | $CH_3$ | N-$OCH_3$ | $OCH_3$ | H |
| 81 | $CH_3CO-$ | 4-$CH_3$-Ph | $CH_3$ | N-$OCH_3$ | $NHCH_3$ | H |
| 82 | $CH_3CO-$ | 4-$CH_3$-Ph | $CH_3$ | CH-$OCH_3$ | $OCH_3$ | H |
| 83 | $CH_3CO-$ | 4-$C_2H_5$-Ph | $CH_3$ | N-$OCH_3$ | $OCH_3$ | H |
| 84 | $CH_3CO-$ | 4-$C_2H_5$-Ph | $CH_3$ | N-$OCH_3$ | $NHCH_3$ | H |
| 85 | $CH_3CO-$ | 4-n-$C_3H_7$-Ph | $CH_3$ | N-$OCH_3$ | $NHCH_3$ | H |
| 86 | $CH_3CO-$ | 3,4-$(CH_3)_2$-Ph | $CH_3$ | N-$OCH_3$ | $OCH_3$ | H |

- Cont'd -

Table 1 (Cont'd)

| No. | $R^2$ | $R^3$ | $R^4$ | X | Y | Zm |
|---|---|---|---|---|---|---|
| 87 | $CH_3CO-$ | 3,4-$(CH_3)_2$-Ph | $CH_3$ | N-$OCH_3$ | $NHCH_3$ | H |
| 88 | $CH_3CO-$ | 2,5-$(CH_3)_2$-Ph | $CH_3$ | N-$OCH_3$ | $OCH_3$ | H |
| 89 | $CH_3CO-$ | 2,5-$(CH_3)_2$-Ph | $CH_3$ | N-$OCH_3$ | $NHCH_3$ | H |
| 90 | $CH_3CO-$ | 4-$CF_3$-Ph | $CH_3$ | N-$OCH_3$ | $OCH_3$ | H |
| 91 | $CH_3CO-$ | 4-$CF_3$-Ph | $CH_3$ | N-$OCH_3$ | $NHCH_3$ | H |
| 92 | $CH_3CO-$ | 2-$CH_3O$-Ph | $CH_3$ | N-$OCH_3$ | $OCH_3$ | H |
| 93 | $CH_3CO-$ | 2-$CH_3O$-Ph | $CH_3$ | N-$OCH_3$ | $NHCH_3$ | H |
| 94 | $CH_3CO-$ | 3-$CH_3O$-Ph | $CH_3$ | N-$OCH_3$ | $OCH_3$ | H |
| 95 | $CH_3CO-$ | 3-$CH_3O$-Ph | $CH_3$ | N-$OCH_3$ | $NHCH_3$ | H |
| 96 | $CH_3CO-$ | 4-$CH_3O$-Ph | $CH_3$ | CH-$OCH_3$ | $OCH_3$ | H |
| 97 | $CH_3CO-$ | 4-$CH_3O$-Ph | $CH_3$ | N-$OCH_3$ | $OCH_3$ | H |
| 98 | $CH_3CO-$ | 4-$CH_3O$-Ph | $CH_3$ | N-$OCH_3$ | $NHCH_3$ | H |
| 99 | $CH_3CO-$ | 2,5-$(CH_3O)_2$-Ph | $CH_3$ | N-$OCH_3$ | $OCH_3$ | H |
| 100 | $CH_3CO-$ | 2,5-$(CH_3O)_2$-Ph | $CH_3$ | N-$OCH_3$ | $NHCH_3$ | H |
| 101 | $CH_3CO-$ | 2,5-$(CH_3O)_2$-Ph | $CH_3$ | CH-$OCH_3$ | $OCH_3$ | H |
| 102 | $CH_3CO-$ | 3,4-$(CH_3O)_2$-Ph | $CH_3$ | N-$OCH_3$ | $OCH_3$ | H |
| 103 | $CH_3CO-$ | 3,4-$(CH_3O)_2$-Ph | $CH_3$ | N-$OCH_3$ | $NHCH_3$ | H |
| 104 | $CH_3CO-$ | 2,3,4-$(CH_3O)_3$-Ph | $CH_3$ | N-$OCH_3$ | $OCH_3$ | H |
| 105 | $CH_3CO-$ | 2,3,4-$(CH_3O)_3$-Ph | $CH_3$ | N-$OCH_3$ | $NHCH_3$ | H |
| 106 | $CH_3CO-$ | 3,4,5-$(CH_3O)_3$-Ph | $CH_3$ | N-$OCH_3$ | $OCH_3$ | H |
| 107 | $CH_3CO-$ | 3,4,5-$(CH_3O)_3$-Ph | $CH_3$ | N-$OCH_3$ | $NHCH_3$ | H |
| 108 | $CH_3CO-$ | 2-i-$C_3H_7O$-Ph | $CH_3$ | N-$OCH_3$ | $OCH_3$ | H |
| 109 | $CH_3CO-$ | 2-i-$C_3H_7O$-Ph | $CH_3$ | N-$OCH_3$ | $NHCH_3$ | H |

- Cont'd -

Table 1 (Cont'd)

| No. | $R^2$ | $R^3$ | $R^4$ | X | Y | Zm |
|---|---|---|---|---|---|---|
| 110 | $CH_3CO-$ | 3-i-$C_3H_7O$-Ph | $CH_3$ | N-$OCH_3$ | $OCH_3$ | H |
| 111 | $CH_3CO-$ | 3-i-$C_3H_7O$-Ph | $CH_3$ | N-$OCH_3$ | $NHCH_3$ | H |
| 112 | $CH_3CO-$ | 4-i-$C_3H_7O$-Ph | $CH_3$ | N-$OCH_3$ | $OCH_3$ | H |
| 113 | $CH_3CO-$ | 4-i-$C_3H_7O$-Ph | $CH_3$ | N-$OCH_3$ | $NHCH_3$ | H |
| 114 | $CH_3CO-$ | 3,4-$OCH_2O$-Ph | $CH_3$ | N-$OCH_3$ | $OCH_3$ | H |
| 115 | $CH_3CO-$ | 3,4-$OCH_2O$-Ph | $CH_3$ | N-$OCH_3$ | $NHCH_3$ | H |
| 116 | $CH_3CO-$ | 4-$CF_3O$-Ph | $CH_3$ | N-$OCH_3$ | $OCH_3$ | H |
| 117 | $CH_3CO-$ | 4-$CHF_2O$-Ph | $CH_3$ | N-$OCH_3$ | $NHCH_3$ | H |
| 118 | $CH_3CO-$ | 4-$CHF_2O$-Ph | $CH_3$ | N-$OCH_3$ | $OCH_3$ | H |
| 119 | $CH_3CO-$ | 4-$CHF_2O$-Ph | $CH_3$ | N-$OCH_3$ | $NHCH_3$ | H |
| 120 | $CH_3CO-$ | 2-$CH_2$=$CHCH_2O$-Ph | $CH_3$ | N-$OCH_3$ | $OCH_3$ | H |
| 121 | $CH_3CO-$ | 2-$CH_2$=$CHCH_2O$-Ph | $CH_3$ | N-$OCH_3$ | $NHCH_3$ | H |
| 122 | $CH_3CO-$ | 3-$CH_2$=$CHCH_2O$-Ph | $CH_3$ | N-$OCH_3$ | $OCH_3$ | H |
| 123 | $CH_3CO-$ | 3-$CH_2$=$CHCH_2O$-Ph | $CH_3$ | N-$OCH_3$ | $NHCH_3$ | H |
| 124 | $CH_3CO-$ | 4-$CH_2$-$CHCH_2O$-Ph | $CH_3$ | N-$OCH_3$ | $OCH_3$ | H |
| 125 | $CH_3CO-$ | 4-$CH_2$-$CHCH_2O$-Ph | $CH_3$ | N-$OCH_3$ | $NHCH_3$ | H |
| 126 | $CH_3CO-$ | 4-Ph-O-Ph | $CH_3$ | N-$OCH_3$ | $OCH_3$ | H |
| 127 | $CH_3CO-$ | 4-Ph-O-Ph | $CH_3$ | N-$OCH_3$ | $NHCH_3$ | H |
| 128 | $CH_3CO-$ | 4-$CH_3S$-Ph | $CH_3$ | N-$OCH_3$ | $OCH_3$ | H |
| 129 | $CH_3CO-$ | 4-$CH_3S$-Ph | $CH_3$ | N-$OCH_3$ | $NHCH_3$ | H |
| 130 | $CH_3CO-$ | 4-$COOCH_3$-Ph | $CH_3$ | N-$OCH_3$ | $OCH_3$ | H |
| 131 | $CH_3CO-$ | 4-$COOC_2H_5$-Ph | $C_2H_5$ | N-$OCH_3$ | $OCH_3$ | H |

- Cont'd -

Table 1 (Cont'd)

| No. | $R^2$ | $R^3$ | $R^4$ | X | Y | Zm |
|---|---|---|---|---|---|---|
| 132 | $CH_3CO-$ | 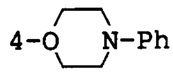 | $CH_3$ | $N-OCH_3$ | $OCH_3$ | H |
| 133 | $CH_3CO-$ | 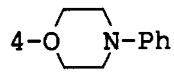 | $CH_3$ | $N-OCH_3$ | $NHCH_3$ | H |
| 134 | $CH_3CO-$ | $Ph-OCH_2$ | $CH_3$ | $N-OCH_3$ | $OCH_3$ | H |
| 135 | $CH_3CO-$ | $Ph-CH=CH$ | $CH_3$ | $N-OCH_3$ | $OCH_3$ | H |
| 136 | $CH_3CO-$ | 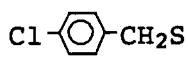 | 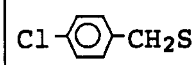 | $N-OCH_3$ | $NHCH_3$ | H |
| 137 | $CH_3CO-$ | 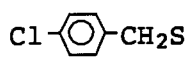 | 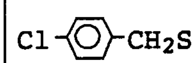 | $N-OCH_3$ | $OCH_3$ | H |
| 138 | $CH_3CO-$ | 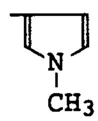 | $CH_3$ | $N-OCH_3$ | $OCH_3$ | H |
| 139 | $CH_3CO-$ | 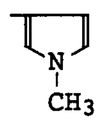 | $CH_3$ | $N-OCH_3$ | $NHCH_3$ | H |
| 140 | $CH_3CO-$ | 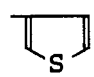 | $CH_3$ | $CH-OCH_3$ | $OCH_3$ | H |
| 141 | $CH_3CO-$ | 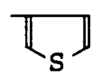 | $CH_3$ | $N-OCH_3$ | $OCH_3$ | H |
| 142 | $CH_3CO-$ | 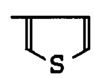 | $CH_3$ | $N-OCH_3$ | $NHCH_3$ | H |
| 143 | $CH_3CO-$ | 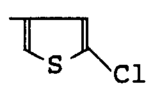 | $CH_3$ | $N-OCH_3$ | $OCH_3$ | H |
| 144 | $CH_3CO-$ | 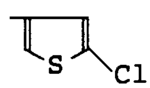 | $CH_3$ | $N-OCH_3$ | $NHCH_3$ | H |

\- Cont'd -

Table 1 (Cont'd)

| No. | $R^2$ | $R^3$ | $R^4$ | X | Y | Zm |
|---|---|---|---|---|---|---|
| 145 | $CH_3CO-$ | 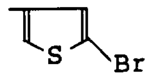 | $CH_3$ | $N-OCH_3$ | $OCH_3$ | H |
| 146 | $CH_3CO-$ | 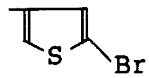 | $CH_3$ | $N-OCH_3$ | $NHCH_3$ | H |
| 147 | $CH_3CO-$ | 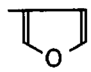 | $CH_3$ | $N-OCH_3$ | $OCH_3$ | H |
| 148 | $CH_3CO-$ | 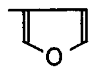 | $CH_3$ | $N-OCH_3$ | $NHCH_3$ | H |
| 149 | $CH_3CO-$ | 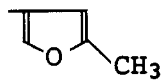 | $CH_3$ | $N-OCH_3$ | $OCH_3$ | H |
| 150 | $CH_3CO-$ | 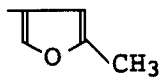 | $CH_3$ | $N-OCH_3$ | $NHCH_3$ | H |
| 151 | $CH_3CO-$ | 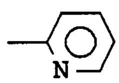 | $CH_3$ | $N-OCH_3$ | $OCH_3$ | H |
| 152 | $CH_3CO-$ | 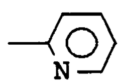 | $CH_3$ | $N-OCH_3$ | $OCH_3$ | H |
| 153 | $CH_3CO-$ | 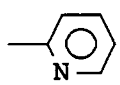 | $CH_3$ | $N-OCH_3$ | $NHCH_3$ | H |
| 154 | $CH_3CO-$ | 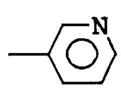 | $CH_3$ | $N-OCH_3$ | $OCH_3$ | H |
| 155 | $CH_3CO-$ | 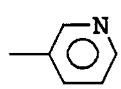 | $CH_3$ | $N-OCH_3$ | $NHCH_3$ | H |
| 156 | $CH_3CO-$ | 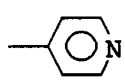 | $CH_3$ | $N-OCH_3$ | $OCH_3$ | H |

- Cont'd -

Table 1 (Cont'd)

| No. | $R^2$ | $R^3$ | $R^4$ | X | Y | Zm |
|---|---|---|---|---|---|---|
| 157 | $CH_3CO-$ | 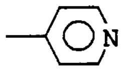 | $CH_3$ | $N-OCH_3$ | $NHCH_3$ | H |
| 158 | $CH_3CO-$ | 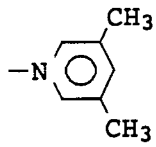 | $CH_3$ | $CH-OCH_3$ | $OCH_3$ | H |
| 159 | $CH_3CO-$ | 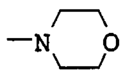 | $CH_3$ | $N-OCH_3$ | $OCH_3$ | H |
| 160 | $CH_3CO-$ | 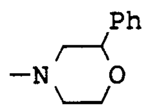 | $CH_3$ | $CH-OCH_3$ | $OCH_3$ | H |
| 161 | $CH_3CO-$ | 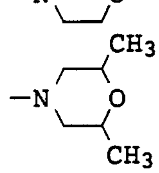 | $CH_3$ | $CH-OCH_3$ | $OCH_3$ | H |
| 162 | $CH_3CO-$ | 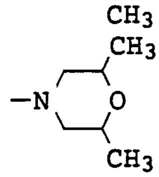 | $CH_3$ | $N-OCH_3$ | $OCH_3$ | H |
| 163 | $CH_3CO-$ | 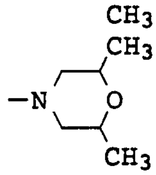 | $CH_3$ | $N-OCH_3$ | $NHCH_3$ | H |
| 164 | $CH_3CO-$ | 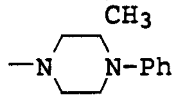 | $CH_3$ | $N-OCH_3$ | $OCH_3$ | H |
| 165 | $CH_3CO-$ | 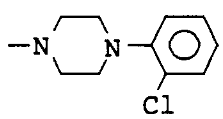 | $CH_3$ | $N-OCH_3$ | $OCH_3$ | H |
| 166 | $CH_3CO-$ | 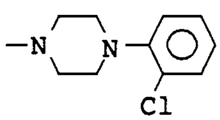 | $CH_3$ | $N-OCH_3$ | $NHCH_3$ | H |
| 167 | $CH_3CO-$ | 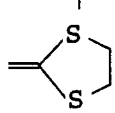 | | $N-OCH_3$ | $OCH_3$ | H |
| 168 | $CH_3CO-$ | 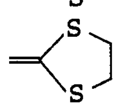 | | $N-OCH_3$ | $NHCH_3$ | H |

- Cont'd -

Table 1 (Cont'd)

| No. | $R^2$ | $R^3$ | $R^4$ | X | Y | Zm |
|---|---|---|---|---|---|---|
| 169 | $C_2H_5CO-$ | Ph | $CH_3$ | $N-OCH_3$ | $OCH_3$ | H |
| 170 | $C_2H_5CO-$ | Ph | $CH_3$ | $N-OCH_3$ | $NHCH_3$ | |
| 171 | Ph-CO- | Ph | $CH_3$ | $N-OCH_3$ | $NHCH_3$ | H |
| 172 | $CH_3SO_2-$ | Ph | $CH_3$ | $N-OCH_3$ | $OCH_3$ | |
| 173 | $CH_3SO_2-$ | Ph | $CH_3$ | $N-OCH_3$ | $NHCH_3$ | H |
| 174 | $CH_3O-CO-$ | Ph | $CH_3$ | $N-OCH_3$ | $OCH_3$ | |
| 175 | $CH_3O-CO-$ | Ph | $CH_3$ | $N-OCH_3$ | $NHCH_3$ | H |
| 176 | $CH_3CO-$ | 4-F-Ph | $C_2H_5$ | $CH-OCH_3$ | $OCH_3$ | |
| 177 | $CH_3CO-$ | $3,4-F_2-P$ | $CH_3$ | $CH-OCH_3$ | $OCH_3$ | H |
| 178 | $CH_3CO-$ | $CH_3S$ | $CH_3$ | $CH-OCH_3$ | $OCH_3$ | |
| 179 | $CH_3CO-$ | 4-F-Ph | $n-C_3H_7$ | $CH-OCH_3$ | $OCH_3$ | H |
| 180 | $CH_3CO-$ | 4-F-Ph | $n-C_5H_{11}$ | $CH-OCH_3$ | $OCH_3$ | |
| 181 | $CH_3CO-$ | $4-n-C_4H_9-Ph$ | $CH_3$ | $CH-OCH_3$ | $OCH_3$ | H |
| 182 | $CH_3CO-$ | 4-F-Ph | $i-C_3H_7$ | $CH-OCH_3$ | $OCH_3$ | |
| 183 | $CH_3CO-$ | 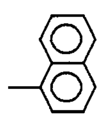 | $CH_3$ | $CH-OCH_3$ | $OCH_3$ | H |
| 184 | $CH_3CO-$ | 4-PhO-Ph | $CH_3$ | $CH-OCH_3$ | $OCH_3$ | H |
| 185 | $CH_3CO-$ | $4-CF_3O-ph$ | $CH_3$ | $CH-OCH_3$ | $OCH_3$ | |
| 186 | $CH_3CO-$ | Ph-CH=CH | $CH_3$ | $N-OCH_3$ | $NHCH_3$ | H |
| 187 | $CH_3CO-$ | 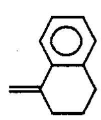 | | $N-OCH_3$ | $OCH_3$ | H |
| 188 | $CH_3CO-$ | 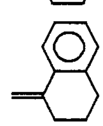 | | $N-OCH_3$ | $NHCH_3$ | H |

- Cont'd -

Table 1 (Cont'd)

| No. | $R^2$ | $R^3$ | $R^4$ | X | Y | Zm |
|---|---|---|---|---|---|---|
| 189 | $CH_3CO-$ | $Ph-CH_2$ | $CH_3$ | $N-OCH_3$ | $OCH_3$ | H |
| 190 | $CH_3CO-$ | $t-C_4H_9$ | $CH_3$ | $N-OCH_3$ | $OCH_3$ | H |
| 191 | $CH_3CO-$ | $n-C_3H_7$ | $CH_3$ | $N-OCH_3$ | $OCH_3$ | H |
| 192 | $CH_3CO-$ | 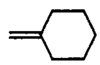 | | $N-OCH_3$ | $OCH_3$ | H |
| 193 | $CH_3CO-$ | Ph | $CH_2SCH_2COOC_2H_5$ | $N-OCH_3$ | $OCH_3$ | H |
| 194 | $CH_3CO-$ | Ph | $CH_2SCH_2COOC_2H_5$ | $N-OCH_3$ | $NHCH_3$ | H |
| 195 | $CH_3CO-$ | $t-C_4H_9$ | $CH_3$ | $N-OCH_3$ | $NHCH_3$ | H |
| 196 | $CH_3CO-$ | $C_2H_5S$ | $C_2H_5S$ | $N-OCH_3$ | $OCH_3$ | H |
| 197 | $CH_3CO-$ | 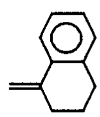 | | $N-OCH_3$ | $OCH_3$ | H |
| 198 | $CH_3CO-$ | 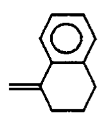 | | $N-OCH_3$ | $NHCH_3$ | H |
| 199 | $CH_3CO-$ | 4-F-Ph | $C_2H_5$ | $N-OCH_3$ | $OCH_3$ | H |
| 200 | $CH_3CO-$ | 4-F-Ph | $n-C_3H_7$ | $N-OCH_3$ | $OCH_3$ | H |
| 201 | $CH_3CO-$ | 4-F-Ph | $i-C_3H_7$ | $N-OCH_3$ | $OCH_3$ | H |
| 202 | $CH_3CO-$ | 4-F-Ph | $n-C_5H_{11}$ | $N-OCH_3$ | $OCH_3$ | H |
| 203 | $CH_3CO-$ | 4-F-Ph | $n-C_3H_7$ | $N-OCH_3$ | $NHCH_3$ | H |
| 204 | $CH_3CO-$ | 4-F-Ph | $i-C_3H_7$ | $N-OCH_3$ | $NHCH_3$ | H |
| 205 | $CH_3CO-$ | 4-F-Ph | $n-C_5H_{11}$ | $N-OCH_3$ | $NHCH_3$ | H |
| 206 | $CH_3CO-$ | $C_2H_5S$ | $C_2H_5S$ | $N-OCH_3$ | $NHCH_3$ | H |

- Cont'd -

Table 1 (Cont'd)

| No. | $R^2$ | $R^3$ | $R^4$ | X | Y | Zm |
|---|---|---|---|---|---|---|
| 207 | $CH_3CO-$ | 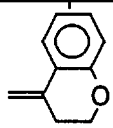 | | $N-OCH_3$ | $OCH_3$ | H |
| 208 | $CH_3CO-$ | 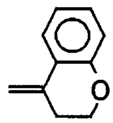 | | $N-OCH_3$ | $NHCH_3$ | H |
| 209 | $CH_3CO-$ | $3,4-F_2Ph$ | $C_2H_5$ | $N-OCH_3$ | $NHCH_3$ | H |
| 210 | $CH_3CO-$ | $4-F-Ph-CH_2S$ | $CH_3S$ | $N-OCH_3$ | $OCH_3$ | H |
| 211 | $CH_3CO-$ | $4-F-Ph-CH_2S$ | $CH_3S$ | $N-OCH_3$ | $NHCH_3$ | H |
| 212 | $CH_3CO-$ | $2,4-Cl_2-PhCH_2S$ | $CH_3S$ | $N-OCH_3$ | $OCH_3$ | H |
| 213 | $CH_3CO-$ | $2,4-Cl_2-PhCH_2S$ | $CH_3S$ | $N-OCH_3$ | $NHCH_3$ | H |

Physical properties of the compounds listed in Table 1 are tabulated in Table 2.

Table 2

| No | Physical property [melting point, refractive index or NMR ($CDCl_3$/TMS, δ values ppm)] |
|---|---|
| 1 | 2.40 (3H, s), 3.87 (3H, s), 4.05 (23H, s), 5.18 (2H, q), 7.18-7.58 (7H, m), 7.82 (2H, dd), 8.28 (1H, s). |
| 2 | m.p. 89-90°C |
| 3 | 1.91 (3H, s), 2.02 (3H, s), 3.35 (2H, s), 3.67 (3H, s), 3.80 (3H, s), 4.01 (3H, s), 5.06 (2H, s), 7.12-7.49 (4H, m), 7.56 (1H, s). |
| 4 | 1.98 (3H, s), 2.02 (3H, s), 3.72 (3H, s), 3.85 (3H, s), 4.03 (3H, s), 5.02 (2H, s), 7.15-7.55 (4H, m). |
| 5 | m.p. 131.6-133.1°C |
| 6 | m.p. 71.3-74.1°C |
| 7 | 2.07 (3H, s), 2.89 (3H, d), 3.6-3.8 (4H, m), 3.95 (3H, s), 5.05 (2H, s), 5.05-5.35 (4H, m), 5.8-6.2 (2H, m), 6.73 (1H, br. q), 7.15-7.6 (4H, m). |
| 8 | m.p. 53.3-54.3°C |
| 9 | nD 1.5672 (13.3°C) |
| 10 | m.p. 84°C |
| 11 | m.p. 110°C |
| 12 | 2.17 (3H, s), 2.34 (3H, s), 3.69 (3H, s), 3.83 (3H, s), 5.18 (2H, s), 7.17-7.55 (7H, m), 7.59 (1H, s), 7.85 (2H, dd). |
| 13 | 1.09 (3H, t), 2.18 (3H, s), 2.89 (2H, q), 3.61 (3H, s), 3.82 (3H, s), 5.16 (2H, s), 7.18-7.56 (7H, m), 7.59 (1H, s), 7.83 (2H, dd). |
| 14 | m.p. 96°C |
| 15 | 1.71 (6H, d), 2.01 (3H, s), 2.88 (1H, m), 2.90 (3H, d), 3.90 (3H, s), 4.70 (2H, s), 6.75 (1H, br. s), 7.06-7.38 (9H, m). |
| 16 | m.p. 156°C |

- Cont'd -

Table 2 (Cont'd)

| No | Physical property [melting point, refractive index or NMR ($CDCl_3$/TMS, δ values ppm)] |
|---|---|
| 17 | m.p. 221°C |
| 18 | m.p. 166°C |
| 19 | 2.10 (3H, s), 2.30 (3H, s), 3.71 (3H, s), 3.84 (3H, s), 5.16 (2H, s), 7.17-7.56 (8H, m), 7.61 (1H, s). |
| 20 | 2.05 (3H, s), 2.34 (3H, s), 3.85 (3H, s), 4.04 (3H, s), 5.12 (2H, s), 7.15-7.55 (8H, m). |
| 21 | 2.06 (3H, s), 2.24 (3H, s), 2.91 (3H, d), 3.96 (3H, s), 5.13 (2H, s), 6.78 (1H, br. q), 7.19-7.55 (8H, m). |
| 22 | 2.19 (3H, s), 2.33 (3H, s), 3.70 (3H, s), 3.83 (3H, s)., 5.17 (2H, s), 7.16-7.55 (6H, m), 7.60 (1H, s), 7.71 (1H, d), 7.84 (1H, s). |
| 23 | m.p. 96°C |
| 24 | 2.13 (3H, s), 2.28 (3H, s), 2.90 (3H, d), 3.96 (3H, s), 5.13 (2H, s), 6.78 (1H, br. q), 7.18-7.83 (8H, m). |
| 25 | m.p. 98°C |
| 26 | m.p. 99°C |
| 27 | m.p. 128°C |
| 28 | m.p. 121°C |
| 29 | m.p. 133°C |
| 30 | m.p. 97°C |
| 31 | m.p. 102°C |
| 32 | 2.06 (3H, s), 2.23 (3H, s), 2.92 (3H, d), 3.96 (3H, s), 5.12 (2H, s), 6.78 (1H, br. q), 7.19-7.55 (7H, m). |
| 33 | m.p. 109°C |
| 34 | m.p. 135°C |
| 35 | m.p. 95°C |

- Cont'd -

Table 2 (Cont'd)

| No | Physical property [melting point, refractive index or NMR ($CDCl_3$/TMS, δ values ppm)] |
|---|---|
| 36 | 2.07 (3H, s), 2.23 (3H, s), 2.92 (3H, d), 3.97 (3H, s), 5.12 (2H, s), 6.89 (1H, br. q), 7.20-7.55 (7H, m). |
| 37 | m.p. 92°C |
| 38 | 2.06 (3H, s), 2.22 (3H, s), 2.93 (3H, d), 3.97 (3H, s), 5.13 (2H, s), 6.88 (1H, br. q), 7.15-7.55 (6H, m). |
| 39 | m.p. 84°C |
| 40 | m.p. 91°C |
| 41 | 2.17 (3H, s), 2.31 (3H, s), 3.76 (3H, s), 3.83 (3H, s), 5.17 (2H, s), 7.13-7.53 (6H, m), 7.58 (1H, s), 7.72 (2H, d). |
| 42 | m.p. 116°C |
| 43 | m.p. 131°C |
| 44 | m.p. 87°C |
| 45 | m.p. 109°C |
| 46 | m.p. 124°C |
| 47 | m.p. 97°C |
| 48 | 2.13 (3H, s), 2.29 (3H, s), 2.91 (3H, d), 3.97 (3H, s), 5.13 (2H, s), 6.77 (1H, br. q), 7.02-7.56 (6H, m), 7.83 (2H, dd). |
| 49 | 1.04 (3H, t), 2.12 (3H, s), 2.83 (2H, l), 2.92 (3H, d), 3.97 (3H, s), 5.12 (3H, s), 6.76 (1H, br. q), 7.03-7.24 (3H, m), 7.36-7.55 (3H, m), 7.76-7.87 (2H, m). |
| 50 | m.p. 79°C |
| 51 | 2.10 (3H, s), 2.29 (3H, d), 2.93 (3H, d), 3.97 (3H, s), 5.13 (2H, s), 6.71-6.95 (3H, m), 7.18-7.72 (5H, m). |
| 52 | m.p. 86°C |
| 53 | m.p. 130°C |

- Cont'd -

Table 2 (Cont'd)

| No | Physical property [melting point, refractive index or NMR ($CDCl_3$/TMS, δ values ppm)] |
|---|---|
| 54 | m.p. 82°C |
| 55 | 2.12 (3H, s), 2.32 (3H, d), 2.93 (3H, d), 3.97 (3H, s), 5.13 (2H, s), 6.77 (1H, br. q), 6.97-7.07 (2H, m), 7.18-7.56 (5H, m). |
| 56 | m.p. 54°C |
| 57 | 2.11 (3H, s), 2.32 (3H, d), 2.93 (3H, d), 3.97 (3H, s), 5.14 (2H, s), 6.78 (1H, br. q), 7.04-7.54 (7H, m). |
| 58 | m.p. 94°C |
| 59 | 2.14 (3H, s), 2.28 (3H, s), 2.92 (3H, d), 3.97 (3H, s), 5.14 (2H, s), 6.73-6.86 (2H, m), 7.20-7.53 (6H, m). |
| 60 | m.p. 76°C |
| 61 | 2.06 (3H, s), 2.22 (3H, s), 2.93 (3H, d), 3.98 (3H, s), 5.13 (2H, s), 6.77 (1H, br. q), 6.93 (2H, t), 7.20-7.55 (6H, m). |
| 62 | m.p. 82°C |
| 63 | m.p. 136°C |
| 64 | m.p. 79°C |
| 65 | m.p. 110°C |
| 66 | m.p. 133°C |
| 67 | 2.14 (3H, s), 2.31 (3H, s), 2.92 (3H, d), 3.97 (3H, s), 5.15 (2H, s), 6.78 (1H, br. q), 7.18-7.57 (6H, m), 7.96 (1H, dd), 8.33 (1H, d). |
| 68 | m.p. 119°C |
| 69 | 2.18 (3H, s), 2.37 (3H, s), 3.68 (3H, s), 3.82 (3H, s), 5.16 (2H, s), 7.13-7.53 (4H, m), 7.58 (1H, d), 7.98 (2H, d), 8.21 (2H, d). |
| 70 | 2.18 (3H, s), 2.36 (3H, s), 3.69 (3H, s), 3.82 (3H, s), 5.18 (2H, s), 7.17-7.54 (4H, m), 7.59 (1H, s), 7.68 (2H, d), 7.95 (2H, d). |

- Cont'd -

Table 2 (Cont'd)

| No | Physical property [melting point, refractive index or NMR ($CDCl_3$/TMS, δ values ppm)] |
|---|---|
| 71 | m.p. 118.9°C |
| 72 | 2.12 (3H, s), 2.29 (3H, s), 2.89 (3H, d), 3.94 (3H, s), 5.13 (2H, s), 6.88 (1H, br. q), 7.15-7.55 (4H, m), 7.66 (2H, d), 7.89 (2H, d). |
| 73 | m.p. 139°C |
| 74 | 2.09 (3H, s), 2.24 (3H, s), 2.40 (3H, s), 3.68 (3H, s), 3.82 (3H, s), 5.13 (2H, s), 7.14-7.56 (8H, m), 7.58 (1H, s). |
| 75 | 2.08 (3H, s), 2.29 (3H, s), 2.39 (3H, s), 3.83 (3H, s), 4.02 (3H, s), 5.14 (2H, s), 7.05-7.55 (8H, m). |
| 76 | 2.06 (3H, s), 2.21 (3H, s), 2.38 (3H, s), 2.90 (3H, d), 3.95 (3H, s), 5.11 (2H, s), 6.72 (1H, br. q), 7.14-7.55 (8H, m). |
| 77 | m.p. 105°C |
| 78 | 2.09 (3H, s), 2.27 (3H, s), 2.37 (3H, s), 2.78 (3H, d), 3.94 (3H, s), 5.11 (2H, s), 6.73 (1H, br.q), 7.14-7.67 (8H, m). |
| 79 | 2.17 (3H, s), 2.33 (3H, s), 2.38 (3H, s), 3.70 (3H, s), 3.83 (3H, s), 5.17 (2H, s), 7.17-7.55 (6H, m), 7.58 (1H, s), 7.76 (2H, d). |
| 80 | m.p. 97°C |
| 81 | m.p. 110°C |
| 82 | m.p. 100°C |
| 83 | m.p. 101°C |
| 84 | m.p. 111°C |
| 85 | m.p. 97°C |
| 86 | m.p. 97°C |
| 87 | m.p. 103°C |
| 88 | m.p. 84°C |

- Cont'd -

Table 2 (Cont'd)

| No | Physical property [melting point, refractive index or NMR ($CDCl_3$/TMS, δ values ppm)] |
|---|---|
| 89 | 2.07 (3H, s), 2.21 (3H, s), 2.32 (3H, s), 2.34 (3H, s), 2.92 (3H, d), 3.97 (3H, s), 5.13 (2H, s), 6.76 (1H, br.q), 7.00-7.55 (7H, m). |
| 90 | m.p. 109°C |
| 91 | m.p. 117°C |
| 92 | m.p. 88°C |
| 93 | 2.08 (3H, s), 2.29 (3H, s), 2.91 (3H, d), 3.84 (3H, s), 3.97 (3H, s), 5.12 (2H, s), 6.77 (1H, br. q), 6.85-7.00 (2H, m), 7.18-7.55 (6H, m). |
| 94 | m.p. 104°C |
| 95 | m.p. 98°C |
| 96 | 2.13 (3H, s), 2.31 (3H, s), 3.68 (3H, s), 3.81 (3H, s), 3.83 (3H, s), 5.13 (2H, s), 6.86 (2H, d), 7.13-7.53 (4H, m), 7.57 (1H, s), 7.79 (2H, s). |
| 97 | m.p. 91°C |
| 98 | m.p. 121°C |
| 99 | m.p. 120°C |
| 100 | 2.07(3H, s), 2.22 (3H, s), 2.91 (3H, d), 3.78 (3H, s), 3.79 (3H, s), 3.96 (3H, s), 5.12 (2H, s), 6.76 (1H, br. q), 6.82-6.92 (2H, m), 6.98 (1H, d), 7.18-7.55 (4H, m). |
| 101 | m.p. 148°C |
| 102 | m.p. 109.5°C |
| 103 | 2.11 (3H, s), 2.26 (3H, s), 2.90 (3H, d), 3.91 (3H, s), 3.92 (3H, s), 3.96 (3H, s), 5.13 (2H, s), 6.75 (1H, br. q), 6.85 (1H, d), 7.15-7.5 (7H, m). |
| 104 | m.p. 95°C |

- Cont'd -

Table 2 (Cont'd)

| No | Physical property [melting point, refractive index or NMR ($CDCl_3$/TMS, δ values ppm)] |
|---|---|
| 105 | 2.07 (3H, s), 2.24 (3H, s), 2.92 (3H, d), 3.88 (6H, s), 3.89 (3H, s), 3.96 (3H, s), 5.12 (2H, s), 6.65 (1H, d), 6.76 (1H, br. q), 7.15-7.55 (4H, m). |
| 106 | m.p. 105°C |
| 107 | m.p. 116°C |
| 108 | m.p. 86°C |
| 109 | 1.32 (3H, s), 1.35 (3H, s), 2.07 (3H, s), 2.22 (3H, s), 2.91 (3H, d), 3.97 (3H, s), 4.59 (1H, q. q), 5.13 (2H, s), 6.78 (1H, br. q), 6.87-6.98 (2H, m), 7.18-7.55 (6H, m). |
| 110 | m.p. 86°C |
| 111 | m.p. 89°C |
| 112 | m.p. 82°C |
| 113 | m.p. 110°C |
| 114 | m.p. 125°C |
| 115 | m.p. 152°C |
| 116 | m.p. 75°C |
| 117 | 2.13 (3H, s), 2.31 (3H, s), 2.92 (3H, d), 3.96 (3H, s), 5.14 (2H, s), 6.76 (1H, br. d), 7.17-7.55 (6H, m), 7.87 (2H, d). |
| 118 | m.p. 113°C |
| 119 | 2.13 (3H, s), 2.29 (3H, s), 2.91 (3H, d), 3.97 (2H, s), 5.14 (2H, s), 6.54 (1H, t), 6.75 (1H, br. q), 7.13 (2H, d), 7.19-7.54 (4H, m), 7.85 (2H, d). |
| 120 | m.p. 98°C |
| 121 | 2.07 (3H, s), 2.24 (3H, s), 2.91 (3H, d), 3.96 (3H, s), 4.51-4.62 (2H, m), 5.12 (2H, s), 5.24-5.43 (2H, m), 5.97-6.11 (1H, m), 6.78 (1H, br. q), 6.89-7.00 (2H, m), 7.20-7.55 (6H, m). |

- Cont'd -

Table 2 (Cont'd)

| No | Physical property [melting point, refractive index or NMR ($CDCl_3$/TMS, δ values ppm)] |
|---|---|
| 122 | m.p. 78°C |
| 123 | m.p. 96°C |
| 124 | m.p. 92°C |
| 125 | m.p. 101°C |
| 126 | 2.11 (3H, s), 2.29 (3H, s), 3.84 (3H, s), 4.03 (3H, s), 5.12 (2H, s), 6.92-7.83 (13H, m). |
| 127 | 2.12 (3H, s), 2.29 (3H, s), 2.90 (3H, d), 3.96 (3H, s), 5.13 (2H, s), 6.74 (1H, br. q), 6.95-7.84 (13H, m). |
| 128 | m.p. 80°C |
| 129 | m.p. 123°C |
| 130 | m.p. 124°C |
| 131 | m.p. 128°C |
| 132 | m.p. 123°C |
| 133 | m.p. 143°C |
| 134 | 2.01, 1.98 (3H, s), 2.08, 2.01 (3H, s), 4.85, 4.83 (3H, s), 4.03, 4.00 (3H, s), 4.80, 4.65 (2H, s), 5.07 (2H, s), 6.79-7.55 (9H, m), a mixture of E-form and Z-form |
| 135 | 2.10 (3H, s), 2.15 (3H, s), 3.86 (3H, s), 4.05 (3H, s), 5.12 (2H, s), 6.99 (2H, s), 7.15-7.56 (9H, m). |
| 136 | m.p. 90.7-94.4°C |
| 137 | m.p. 126-128°C |
| 138 | 2.09 (3H, s), 2.17 (3H, s), 3.66 (3H, s), 3.82 (3H, s), 4.01 (3H, s), 4.86 (2H, s), 6.55-7.55 (7H, m). |
| 139 | 2.07 (3H, s), 2.13 (3H, s), 2.87 (3H, d), 3.65 (3H, s), 3.94 (3H, s), 5.08 (2H, s), 6.75 (1H, br. q), 6.55-7.55 (7H, m). |

- Cont'd -

Table 2 (Cont'd)

| No | Physical property [melting point, refractive index or NMR ($CDCl_3$/TMS, δ values ppm)] |
|---|---|
| 140 | 2.14 (3H, s), 2.33 (3H, s), 3.67 (3H, s), 3.81 (3H, s), 5.13 (2H, s), 7.02 (1H, dd), 7.12-7.53 (6H, m), 7.57 (1H, s). |
| 141 | m.p. 77°C |
| 142 | 2.10 (3H, s), 2.29 (3H, s), 2.88 (3H, d), 3.93 (3H, s), 5.10 (2H, s), 6.72 (1H, br. q), 7.01 (1H, t), 7.16-7.51 (6H, m). |
| 143 | m.p. 71°C |
| 144 | m.p. 148°C |
| 145 | m.p. 89°C |
| 146 | m.p. 146°C |
| 147 | m.p. 95.3°C |
| 148 | 2.12 (3H, s), 2.20 (3H, s), 2.89 (3H, d), 3.95 (3H, s), 5.11 (2H, s), 6.42 (1H, dd), 6.72 (1H, br. q), 6.73 (1H, d), 7.20 (1H, d), 7.30-7.55 (4H, m). |
| 149 | m.p. 110°C |
| 150 | 2.11 (3H, s), 2.15 (3H, s), 2.36 (3H, s), 2.90 (3H, d), 3.95 (3H, s), 5.10 (2H, s), 6.06 (1H, d), 6.64 (1H, d), 6.72 (1H, br. q), 7.15-7.55 (4H, m). |
| 151 | m.p. 105°C |
| 152 | m.p. 91°C |
| 153 | 2.23 (3H, s), 2.43 (3H, s), 2.91 (3H, d), 3.97 (3H, s), 5.17 (2H, s), 6.78 (1H, br. q), 7.16-7.48 (4H, m), 7.53 (1H, d), 7.70 (1H, t), 8.17 (1H, d), 8.62 (1H, d). |
| 154 | m.p. 101°C |
| 155 | 2.14 (3H, s), 2.32 (3H, s), 2.92 (3H, d), 3.96 (3H, s), 5.15 (2H, s), 6.77 (1H, br. q), 7.17-7.55 (5H, m), 8.12 (1H, m), 8.60 (1H, m), 9.03 (1H, m). |

- Cont'd -

Table 2 (Cont'd)

| No | Physical property [melting point, refractive index or NMR ($CDCl_3$/TMS, δ values ppm)] |
|---|---|
| 156 | 2.15 (3H, s), 2.30 (3H, s), 3.86 (3H, s), 4.05 (3H, s), 5.15 (2H, s), 7.18-7.55 (4H, m), 7.68 (2H, d), 8.63 (2H, d). |
| 157 | 2.15 (3H, s), 2.29 (3H, s), 2.92 (3H, d), 3.96 (3H, s), 5.16 (2H, s), 6.77 (1H, br. q), 7.18-7.55 (4H, m), 7.68 (2H, d), 8.63 (2H, d). |
| 158 | 0.89 (6H, d), 2.07 (3H, s), 2.10 (3H, s), 2.17 (2H, dd), 3.60-4.05 (6H, m), 3.68 (3H, s), 3.80 (3H, s), 5.03 (2H, s), 7.13-7.51 (4H, m), 7.54 (1H, s). |
| 159 | 2.03 (3H, s), 2.08 (3H, s), 3.36 (4H, t), 3.72 (4H, t), 3.85 (3H, s), 4.02 (3H, s), 5.03 (2H, s), 7.18-7.50 (4H, m). |
| 160 | 2.06 (3H, s), 2.13 (3H, s), 2.75-3.08 (2H, m), 3.68 (3H, s), 3.82 (3H, s), 3.64-4.10 (4H, m), 4.52 (1H, dd), 5.03 (2H, s), 7.10-7.51 (9H, m), 7.56 (1H, s). |
| 161 | 1.19 (6H, d), 2.02 (3H, s), 2.05 (3H, s), 2.41 (2H, dd), 3.52-3.83 (4H, m), 3.65 (3H, s), 3.76 (3H, s), 5.02 (2H, s), 7.08-7.50 (4H, m), 7.53 (1H, s). |
| 162 | m.p. 75°C |
| 163 | 1.17 (6H, d), 1.99 (3H, s), 2.00 (3H, s), 2.40 (2H, m), 2.87 (3H, d), 3.50-3.85 (4H, br. m), 3.91 (3H, s), 4.98 (2H, s), 6.69 (1H, br. q), 7.10-7.50 (4H, m). |
| 164 | m.p. 129°C |
| 165 | m.p. 140.1°C |
| 166 | 2.09 (3H, s), 2.18 (3H, s), 2.91 (3H, d), 3.08 (4H, br. t), 3.54 (4H, br. t), 3.59 (3H, s), 5.03 (2H, s), 6.73 (1H, br. q), 6.95-7.55 (8H, m). |
| 167 | m.p. 122.9-125.0°C |

- Cont'd -

Table 2 (Cont'd)

| No | Physical property [melting point, refractive index or NMR ($CDCl_3$/TMS, δ values ppm)] |
|---|---|
| 168 | m.p. 121-124.0°C |
| 169 | m.p. 84°C |
| 170 | m.p. 138°C |
| 171 | m.p. 143°C |
| 172 | m.p. 158°C |
| 173 | m.p. 143°C |
| 174 | m.p. 134°C |
| 175 | 1.98 (3H, s), 2.76 (3H, d), 3.73 (3H, s), 3.93 (3H, s), 4.74 (2H, s), 6.81 (1H, br. q), 7.14-7.50 (7H, m), 7.76 (2H, d). |
| 176 | m.p. 88°C |
| 177 | m.p. 114°C |
| 178 | nD 1.5894 (23.6°C) |
| 179 | 0.92 (3H, t), 1.52 (2H, m), 2.19 (3H, s), 2.87 (2H, t), 3.70 (3H, s), 3.83 (3H, s), 5.15 (2H, s), 7.18 (1H, m), 7.36 (2H, m), 7.53 (2H, m), 7.59 (1H, s), 7.82 (2H, dd). |
| 180 | 0.88 (3H, t), 1.25-1.50 (6H, m), 2.18 (3H, s), 2.88 (2H, t), 3.70 (3H, s), 3.82 (3H, s), 5.14 (2H, s), 7.07 (2H, t), 7.17-7.55 (4H, m), 7.58 (1H, s), 7.82 (2H, dd). |
| 181 | 0.93 (3H, t), 1.25-1.68 (4H, m), 2.17 (3H, s), 2.32 (3H, s), 2.64 (2H, t), 3.69 (3H, s), 3.83 (3H, s), 5.17 (2H, s), 7.17-7.81 (8H, m), 7.59 (1H, s). |
| 182 | 1.18 (6H, d), 2.10 (3H, s), 2.90 (1H, m), 3.65 (3H, s), 3.69 (3H, s), 4.73 (2H, s), 6.97-7.35 (8H, m), 7.47 (1H, s). |
| 183 | 2.17 (3H, s), 2.44 (3H, s), 3.72 (3H, s), 3.87 (3H, s), 5.21 (2H, s), 7.18-7.58 (8H, m), 7.63 (1H, s), 7.87 (2H, m), 8.20 (1H, m). |

- Cont'd -

Table 2 (Cont'd)

| No | Physical property [melting point, refractive index or NMR ($CDCl_3$/TMS, δ values ppm)] |
|---|---|
| 184 | 2.18 (3H, s), 2.33 (3H, s), 3.70 (3H, s), 3.83 (3H, s), 5.16 (2H, s), 6.98-7.86 (14H, m). |
| 185 | 2.18 (3H, s), 2.34 (3H, s), 3.70 (3H, s), 3.83 (3H, s), 5.18 (2H, s), 7.13-7.91 (9H, m). |
| 186 | m.p. 146°C |
| 187 | m.p. 97°C |
| 188 | 1.85-1.95 (2H, m), 2.13 (3H, s), 2.70-2.85 (4H, m), 2.90 (3H, d), 3.96 (3H, s), 5.12 (2H, s), 8.75 (1H, br. q), 7.10-7.55 (7H, m), 8.22 (1H, d). |
| 189 | 1.88 (3H, s), 1.92 (3H, s), 2.55-2.97 (4H, m), 3.84 (3H, s), 4.02 (3H, s), 5.04 (2H, s), 7.12-7.50 (9H, m). |
| 190 | nD 1.5209 (23.7°C) |
| 191 | nD 1.5293 (20.7°C) |
| 192 | 1.10-1.20 (2H, br. m), 1.40-1.50 (2H, br. m), 1.55-1.65 (2H, br. m), 1.81 (3H, s), 1.90-2.00 (2H, br. m), 2.20-2.30 (2H, br. m), 3.80 (3H, s), 3.97 (3H, s), 4.50 (2H, br), 7.00-7.45 (4H, m). |
| 193 | 1.22 (3H, t), 2.19 (3H, s), 3.20 (3H, s), 3.87 (3H, s), 4.06 (3H, s), 4.09 (2H, s), 4.08 (2H, q), 5.16 (2H, s), 7.20-8.00 (9H, m). |
| 194 | nD 1.5780 (16.8°C) |
| 195 | m.p. 108°C |
| 196 | m.p. 99°C |
| 197 | m.p. 99°C |
| 198 | m.p. 125°C |
| 199 | m.p. 100°C |
| 200 | m.p. 110°C |
| 201 | m.p. 91°C |

- Cont'd -

Table 2 (Cont'd)

| No | Physical property [melting point, refractive index or NMR ($CDCl_3$/TMS, δ values ppm)] |
|---|---|
| 202 | m.p. 79°C |
| 203 | m.p. 109°C |
| 204 | 1.17 (6H, d), 2.06 (3H, s), 2.82-2.94 (4H, m), 3.82 (3H, s), 4.72 (2H, s), 6.68 (1H, br. q), 6.95-7.34 (8H, m). |
| 205 | 0.90 (3H, t), 1.26-1.51 (6H, m), 2.14 (3H, s), 2.86 (2H, m), 2.92 (3H, d), 3.96 (3H, s), 5.11 (2H, s), 6.76 (1H, br. q), 7.06 (2H, t), 7.19-7.55 (4H, m), 7.81 (2H, dd). |
| 206 | nD 1.5727 (11.3°C) |
| 207 | m.p. 108°C |
| 208 | m.p. 150°C |
| 209 | m.p. 95°C |
| 210 | m.p. 108°C |
| 211 | m.p. 122°C |
| 212 | m.p. 116.5°C |
| 213 | m.p. 110.5°C |

The compound of the general formula (II), i.e., the starting compound for producing the benzylhydrazone derivative of the general formula (I) of the present invention can be produced by any of the production processes disclosed in Japanese Patent Unexamined Publication Nos. 4-288045, 4-261147, 4-182461, 3-169842 and 3-120246, etc.

The compound of the general formula (III) can be produced by the process described in A. Maquestiau, Tetrahedron, 1987, 4185, etc.

Typical examples of the present invention are described below, but they should not be construed as limiting the scope of the invention.

Example 1

Production of methyl 2-[2-[1-acetyl-2-[1-(3-chlorophenyl)ethylidene]-1-hydrazinomethyl]phenyl]-2-methoxypropenoate (compound No. 22)

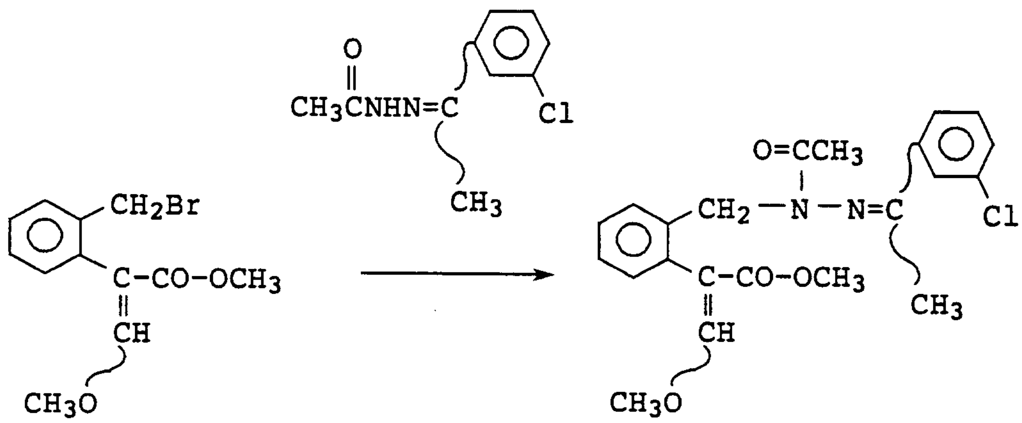

To 20 ml of dimethylformamide were added 0.15 g (3.7 mmoles, purity 62%) of sodium hydride and then 0.78 g (3.7 mmoles) of 2-[1-(3-chlorophenyl)ethylidene]-acetohydrazide, and the resulting mixture was stirred at room temperature for 30 minutes. Then, 0.89 g (3.1 mmoles) of methyl 2-(2-bromomethylphenyl)-3-methoxypropenoate was added to the mixture and the reaction was carried out with stirring for 3 hours.

After completion of the reaction, the reaction mixture was poured into water and the desired compound was extracted with ethyl acetate. The extracted solution was washed with water and dried over anhydrous sodium sulfate, after which the solvent was distilled off under reduced pressure. The crude product thus obtained was purified by a column chromatography (ethyl acetate : n-hexane = 1 : 2) to obtain 0.73 g of the desired compound.

Physical property: paste. Yield: 57%.

$H^1$-NMR [$CDCl_3$/TMS, $\delta$ values (ppm)]

2.19 (3H, s), 2.33 (3H, s), 3.70 (3H, s), 3.83 (3H, s), 5.11 (2H, s), 7.16-7.55 (6H, m), 7.60 (1H, s), 7.71 (1H, d), 7.84 (1H, s).

Example 2

Production of methyl 2-[2-[1-acetyl-2-[1-(4-methylphenyl)ethylidene]hydrazinomethyl]phenyl]-2-methoxyiminoacetate (compound No. 80)

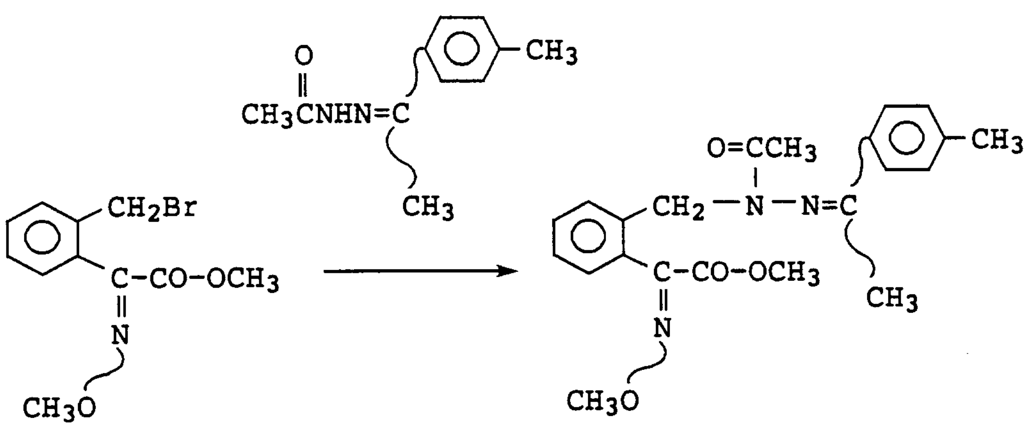

To 20 ml of dimethylformamide were added 0.11 g (2.8 mmoles, purity 62%) of sodium hydride and then 0.50 g (2.6 mmoles) of 2-[1-(4-methylphenyl)ethylidene]-acetohydrazide, and the resulting mixture was stirred at room temperature for 30 minutes. Then, 1.0 g (2.8 mmoles, purity 80%) of methyl 2-(2-bromomethylphenyl)-2-methoxyiminoacetate was added to the mixture and the reaction was carried out with stirring for 4 hours.

After completion of the reaction, the reaction mixture was poured into water and the desired compound was extracted with ethyl acetate. The extracted solution was washed with water and dried over anhydrous sodium sulfate, after which the solvent was distilled off under reduced pressure. The crude product thus obtained was purified by a column chromatography (ethyl acetate : n-hexane = 1 : 2) to obtain 0.30 g of the desired compound.

Physical property: m.p. 97 °C. Yield: 29%.

Example 3

Production of N-methyl-2-[2-[1-acetyl-2-[1-(3,4-dimethoxyphenyl)ethylidene]hydrazinomethyl]-phenyl]-3-methoxyiminoacetamide (compound No. 103)

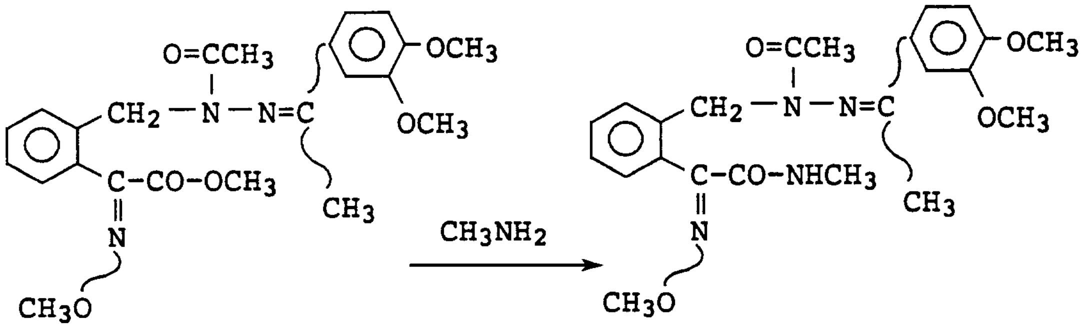


In 30 ml of methanol was dissolved 0.30 g (0.68 mmole) of methyl 2-[2-[1-acetyl-2-[1-(3,4-dimethoxyphenyl)ethylidene]hydrazinomethyl]-phenyl]-3-methoxyiminoacetate, after which 2 ml of a 40% aqueous methylamine solution was added to the resulting solution, and the reaction was carried out at room temperature for 2 hours.

After completion of the reaction, the methanol was distilled off under reduced pressure and the desired compound was extracted with ethyl acetate. The extracted solution was washed with water and dried over anhydrous sodium sulfate, after which the solvent was distilled off under reduced pressure to obtain 0.30 g of the desired compound.

Physical property: paste. Yield: 100%.

$H^1$-NMR [$CDCl_3$/TMS, $\delta$ values (ppm)]

2.11 (3H, s), 2.26 (3H, s), 2.90 (3H, d), 3.91 (3H, s), 3.92 (3H, s), 3.96 (3H, s), 5.13 (2H, s), 6.75 (1H, br. q), 6.85 (1H, d), 7.15-7.56 (7H, m).

Example 4

Production of N-methyl-2-[2-[1-acetyl-2-[1-(4-phenyl-1-piperazinyl)ethylidene]hydrazinomethyl]-phenyl]-3-methoxyiminoacetamide (compound No. 164)

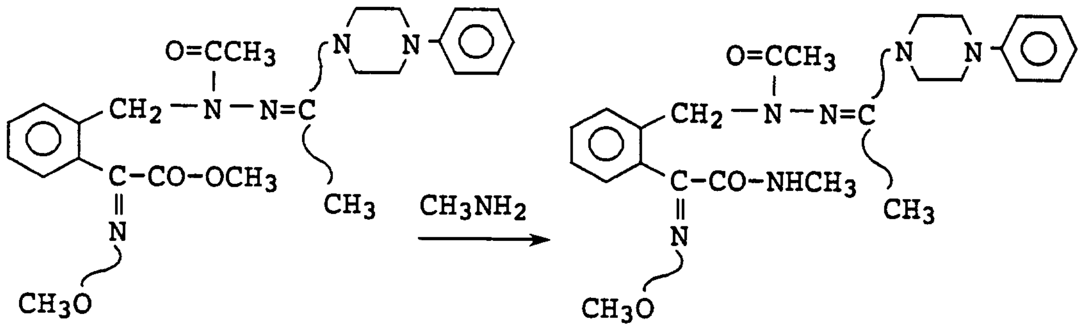


In 30 ml of methanol was dissolved 0.20 g (0.42 mmole) of methyl 2-[2-[1-acetyl-2-[1-(4-phenyl-1-piperazinyl)ethylidene]hydrazinomethyl]phenyl]-3-methoxyiminoacetate, after which 2 ml of a 40% aqueous methylamine solution was added to the resulting solution, and the reaction was carried out at room temperature for 2 hours.

After completion of the reaction, the methanol was distilled off under reduced pressure and the desired compound was extracted with ethyl acetate. The extracted solution was washed with water and dried over anhydrous sodium sulfate, after which the solvent was distilled off under reduced pressure to obtain 0.20 g of the desired compound.

Physical property: m.p. 129 ° C. Yield: 100%.

Example 5

Production of methyl 2-[2-[1-acetyl-2-(1-morpholinoethylidene)hydrazinomethyl]phenyl]-2-methoxyiminoacetate (compound No. 159)

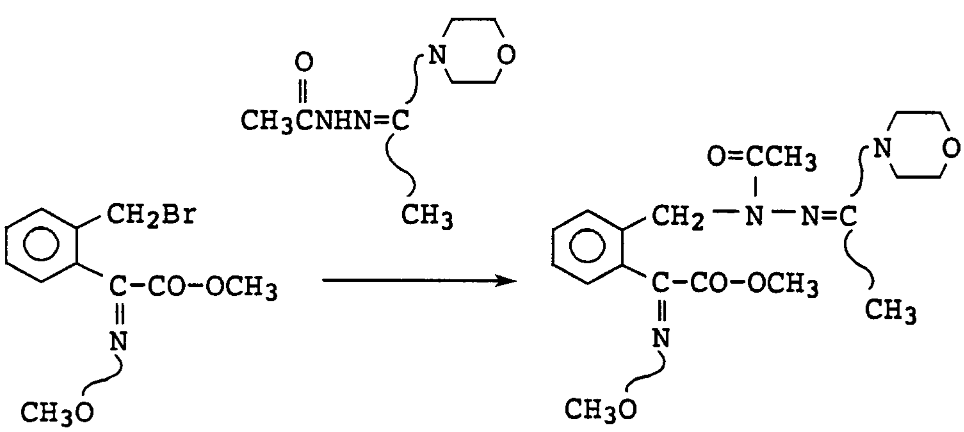

To 60 ml of dimethylformamide were added 0.60 g (5.4 mmoles) of potassium t-butoxide and then 0.80 g (4.2 mmoles) of 2-(1-morpholinoethylidene)acetohydrazide, and the resulting mixture was stirred at room temperature for 30 minutes. Then, the mixture was cooled to 0 ° C and 1.2 g (4.2 mmoles) of methyl 2-(2-bromomethylphenyl)-2-methoxyiminoacetate was added, after which the reaction was carried out with stirring for 3 hours.

After completion of the reaction, the reaction mixture was poured into water and the desired compound was extracted with ethyl acetate. The extracted solution was washed with water and dried over anhydrous sodium sulfate, after which the solvent was distilled off under reduced pressure. The crude product thus obtained was purified by a column chromatography (ethyl acetate : n-hexane = 1 : 2) to obtain 0.66 g of the desired compound.

Physical property: paste. Yield: 40%.

$H^1$-NMR [$CDCl_3$/TMS, δ values (ppm)]

2.03 (3H, s), 2.08 (3H, s), 3.36 (4H, t), 3.72 (4H, t), 3.85 (3H, s), 4.02 (3H, s), 5.03 (2H, s), 7.18-7.50 (4H, m).

Example 6

Production of methyl 2-[2-[1-acetyl-2-[1-(2,6-dimethylmorpholino)ethylidene]hydrazinomethyl]-phenyl]-3-methoxypropenoate (compound No. 161)

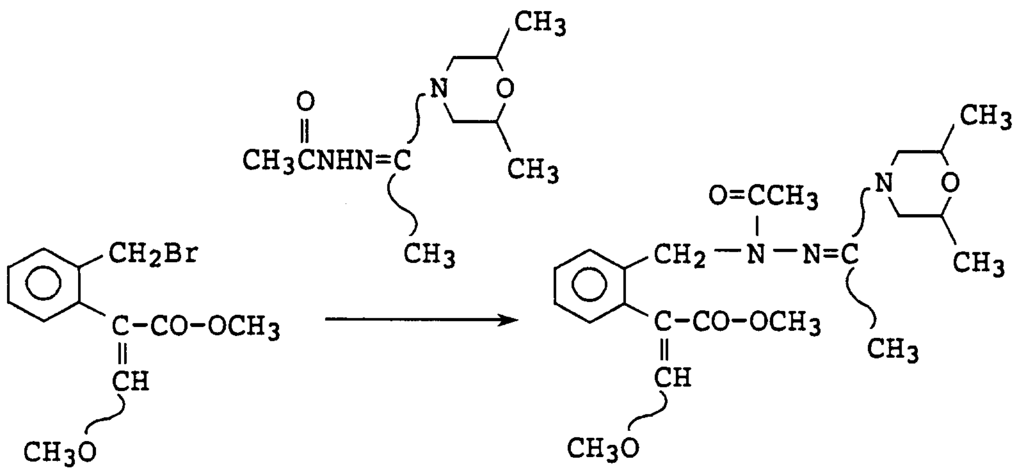

To 60 ml of dimethylformamide were added 0.90 g (7.9 mmoles) of potassium t-butoxide and then 1.7 g (7.9 mmoles) of 2-[1-(2,6-dimethylmorpholino)-ethylidene]acetohydrazide, and the resulting mixture was stirred at room temperature for 30 minutes. Then, the mixture was cooled to 0 ° C and 1.5 g (5.3 mmoles) of methyl 2-(2-bromomethylphenyl)-3-methoxypropenoate was added, after which the reaction was carried out with stirring for 3 hours.

After completion of the reaction, the reaction mixture was poured into water and the desired compound was extracted with ethyl acetate. The extracted solution was washed with water and dried over anhydrous sodium sulfate, after which the solvent was distilled off under reduced pressure. The crude product thus obtained was purified by a column chromatography (ethyl acetate : n-hexane = 1 : 2) to obtain 0.48 g of the desired compound.

Physical property: paste. Yield: 22%.

$H^1$-NMR [$CDCl_3$/TMS, δ values (ppm)]

1.19 (6H, d), 2.02 (3H, s), 2.05 (3H, s), 2.41 (2H, dd), 3.52-3.83 (4H, m), 3.65 (3H, s), 3.76 (3H, s), 5.02 (2H, s), 7.08-7.50 (4H, m), 7.53 (1H, s).

The benzylhydrazone derivatives of the general formula (I) of the present invention are useful as agricultural and horticultural fungicides and are very effective in controlling various diseases, for example, rice blast (Pyricularia oryzae), rice sheath blight (Rhizoctonia solani), rice helminthosporium leaf spot (Cochliobolus miyabeanus), powdery mildew of various host plants, such as powdery mildew of barley and wheat (Erysiphe graminis), oats crown rust (Puccinia coronata), rust of other plants, tomato late blight (Phytophthora infestans), late blight or Phytophthora rots of other plants, downy mildew of various plants, such as cucumber downy mildew (Pseudoperonospora cubensis) and grape downy mildew (Plasmopara viticola), gray mold of cucumber and other plants (Botrytis cinerea), apple scab (Venturia inaequalis), apple alternaria leaf spot (Alternaria mali), pear black spot (Alternaria kikuchiana), and citrus melanose (Diaporthe citri).

The agricultural and horticultural fungicide of the present invention has a marked fungicidal effect on the above-exemplified diseases which damage paddy field crops, upland crops, fruit trees, vegetables, other crops, flowers and ornamental plants, and the like. Therefore, the desired effects of the agricultural and horticultural fungicide of the present invention can be obtained by applying the fungicide to the paddy field

water, seeds, stalks and leaves of fruit trees, vegetables, other crops, flowers and ornamental plants, soil, etc., at a season at which the diseases are expected to occur, before their occurrence or at the time when their occurrence is confirmed.

When the benzylhydrazone derivative of the general formula (I) of the present invention is used as an agricultural and horticultural fungicide, it is generally prepared into conveniently usable forms according to an ordinary manner for preparation of agrochemicals.

That is, the benzylhydrazone derivative of the general formula (I) of the present invention and, optionally, an adjuvant are blended with a suitable inert carrier in a proper proportion and prepared into a suitable preparation form such as a suspension, emulsifiable concentrate, soluble concentrate, wettable powder, granules, dust or tablets through dissolution, dispersion, suspension, mixing, impregnation, adsorption or sticking.

The inert carrier used in the present invention may be either solid or liquid. As the solid carrier, there can be exemplified soybean flour, cereal flour, wood flour, bark flour, saw dust, powdered tobacco stalks, powdered walnut shells, bran, powdered cellulose, extraction residue of vegetables, powdered synthetic polymers or resins, clays (e.g. kaolin, bentonite, and acid clay), talcs (e.g. talc and pyrophyllite), silica powders or flakes (e.g. diatomaceous earth, silica sand, mica and white carbon, i.e. synthetic, high-dispersion silicic acid, also called finely divided hydrated silica or hydrated silicic acid, some of commercially available products contain calcium silicate as the major component), activated carbon, powdered sulfur, powdered pumice, calcined diatomaceous earth, ground brick, fly ash, sand, calcium carbonate powder, calcium phosphate powder and other inorganic or mineral powders, chemical fertilizers (e.g. ammonium sulfate, ammonium phosphate, ammonium nitrate, urea and ammonium chloride), and compost. These carriers may be used alone or as a mixture thereof.

The liquid carrier is that which itself has solubility or which is without such solubility but is capable of dispersing an active ingredient with the aid of an adjuvant. The following are typical examples of the liquid carrier and can be used alone or as a mixture thereof. Water; alcohols such as methanol, ethanol, isopropanol, butanol and ethylene glycol; ketones such as acetone, methyl ethyl ketone, methyl isobutyl ketone, diisobutyl ketone and cyclohexanone; ethers such as ethyl ether, dioxane, Cellosolve, dipropyl ether and tetrahydrofuran; aliphatic hydrocarbons such as kerosene and mineral oils; aromatic hydrocarbons such as benzene, toluene, xylene, solvent naphtha and alkylnaphthalenes; halogenated hydrocarbons such as dichloroethane, chloroform, carbon tetrachloride and chlorobenzene; esters such as ethyl acetate, diisopropyl phthalate, dibutyl phthalate and dioctyl phthalate; amides such as dimethylformamide, diethylformamide and dimethylacetamide; nitriles such as acetonitrile; and dimethyl sulfoxide.

The following are typical examples of the adjuvant, which are used depending upon purposes and used alone or in combination in some cases, or need not to be used at all.

To emulsify, disperse, dissolve and/or wet an active ingredient, a surfactant is used. As the surfactant, there can be exemplified polyoxyethylene alkyl ethers, polyoxyethylene alkylaryl ethers, polyoxyethylene higher fatty acid esters, polyoxyethylene resinates, polyoxyethylene sorbitan monolaurate, polyoxyethylene sorbitan monooleate, alkylarylsulfonates, naphthalenesulfonic acid condensation products, ligninsulfonates and higher alcohol sulfate esters.

Further, to stabilize the dispersion of an active ingredient, tackify it and/or bind it, there may be used adjuvants such as casein, gelatin, starch, methyl cellulose, carboxymethyl cellulose, gum arabic, polyvinyl alcohols, turpentine, bran oil, bentonite and ligninsulfonates.

To improve the flowability of a solid product, there may be used adjuvants such as waxes, stearates and alkyl phosphates.

Adjuvants such as naphthalenesulfonic acid condensation products and polycondensates of phosphates may be used as a peptizer for dispersible products.

Adjuvants such as silicon oils may also be used as a defoaming agent.

The content of the active ingredient may be varied as required. In dusts or granules, the suitable content thereof is from 0.01 to 50% by weight. In emulsifiable concentrates or flowable wettable powders, it is also from 0.01 to 50% by weight.

The agricultural and horticultural fungicide containing the benzylhydrazone derivative of the general formula (I) of the present invention as an active ingredient is used to control various diseases in the following manner. That is, it is applied to a crop on which the diseases are expected to occur, or a site where the occurrence of the diseases is undesirable, as it is or after being properly diluted with or suspended in water or the like, in an amount effective for control of the diseases.

The applying dosage of the agricultural and horticultural fungicide containing the benzylhydrazone derivative of the general formula (I) of the present invention as an active ingredient is varied depending upon various factors such as a purpose, diseases to be controlled, a growth state of a plant, tendency of

disease occurrence, weather, environmental conditions, a preparation form, an application method, an application site and application time. It may be properly chosen in the range of 0.1 g to 1 kg (in terms of the active ingredient) per 10 ares depending upon purposes.

The agricultural and horticultural fungicide containing the benzylhydrazone derivative of the general formula (I) of the present invention as an active ingredient may be used in admixture with other agricultural and horticultural fungicides in order to expand both spectrum of controllable diseases and the period of time when effective applications are possible or to reduce the dosage.

Typical preparation examples and test examples of the present invention are described below but they should not be construed as limiting the scope of the invention.

In the preparation examples, parts are all by weight.

Formulation Example 1

| | |
|---|---|
| Each compound of the invention | 50 parts |
| Xylene | 40 parts |
| Mixture of polyoxyethylene nonylphenyl ether and calcium alkylbenzenesulfonate | 10 parts |

An emulsifiable concentrate was prepared by mixing uniformly the above ingredients to effect dissolution.

Formulation Example 2

| | |
|---|---|
| Each compound of the invention | 3 parts |
| Clay powder | 82 parts |
| Diatomaceous earth powder | 15 parts |

A dust was prepared by mixing uniformly and grinding the above ingredients.

Formulation Example 3

| | |
|---|---|
| Each compound of the invention | 5 parts |
| Mixed powder of bentonite and clay | 90 parts |
| Calcium lignin sulfonate | 5 parts |

Granules were prepared by mixing the above ingredients uniformly, and kneading the resulting mixture together with a suitable amount of water, followed by granulation and drying.

Formulation Example 4

| | |
|---|---|
| Each compound of the invention | 20 parts |
| Mixture of kaolin and synthetic, high-dispersion silicic acid | 75 parts |
| Mixture of polyoxyethylene nonylphenyl ether and calcium alkylbenzenesulfonate | 5 parts |

A wettable powder was prepared by mixing uniformly and grinding the above ingredients.

Test Example 1

Controlling effect on barley powdery mildew

Potted barley plants at the 1 leaf stage were inoculated with spores of powdery mildew fungus (Erysiphe graminis f. sp. hordei) by sprinkling. After 24 hours, they were sprayed with a 200 ppm liquid chemical containing each compound of the present invention as active ingredient, and then allowed to stand in a room thermostated at 25 °C.

One week after the inoculation, the lesion area of each leaf was measured and then compared with that on the untreated plot, whereby the effect was judged according to the following criterion.

| Effect | Controlling degree (%) |
| --- | --- |
| A | 100 - 95 |
| B | 94 - 80 |
| C | 79 - 60 |
| D | 59 - 0 |

The results obtained are shown in Table 3.

Test Example 2

Controlling effect on cucumber downy mildew

Potted cucumber plants at the 2 leaf stage were sprayed with a 200 ppm liquid chemical containing each compound of the present invention as active ingredient. After 24 hours, they were inoculated with a suspension of zoospores of downy mildew fungus (Pseudoperonospora cubensis) by spraying.

After the inoculation, the plants were placed in a moist chamber at 25 °C for 1 day and then a greenhouse for 6 days to cause the disease sufficiently. Thereafter, the degree of occurrence of the disease in each leaf was investigated by comparison with that on the untreated plot, and the effect was judged according to the same criterion as described in Test Example 1.

The results obtained are shown in Table 3.

Test Example 3

Controlling effect on rice blast by spraying

Potted rice plants at the 5 leaf stage were sufficiently sprayed with a 200 ppm liquid chemical containing each compound of the present invention as active ingredient. After air-drying, the plants were inoculated with a suspension of spores of blast fungus (Pyricularia oryzae) by spraying.

After the inoculation, the plants were placed in a moist chamber at 20 °C for 1 day and then a greenhouse for 6 days to cause the disease sufficiently. Then, lesions in each leaf were counted and then compared with those on the untreated plot, whereby the effect was judged according to the same criterion as described in Test Example 1.

The results obtained are shown in Table 3.

Test Example 4

Controlling effect on tomato late blight

Potted tomato plants at the 4 leaf stage were sprayed with a 200 ppm liquid chemical containing each compound of the present invention as active ingredient. After 24 hours, they were inoculated with a suspension of zoospores of late blight fungus (Phytophthora infestans) by spraying. The plants were placed in a moist chamber at 25 °C for 1 day and then a greenhouse for 6 days to cause the disease sufficiently. Thereafter, the degree of occurrence of the disease in each leaf was investigated and then compared with that on the untreated plot, whereby the effect was judged according to the same criterion as described in Test Example 1.

The results obtained are shown in Table 3.

Test Example 5

Controlling effect (curative effect) on cucumber gray mold

The cotyledons of potted cucumber plants at the 1 leaf stage were cut off and each of them was inoculated with a mycelial tuft of gray mold fungus (Botrytis cinerea) cultured on PSA medium. After having been placed in a moist chamber at 15 °C for 24 hours, the cotyledons were immersed in a 200 ppm liquid chemical containing each compound of the present invention as active ingredient.

Then, the cotyledons were placed in a moist chamber at 15 °C for 3 days to cause the disease sufficiently, after which the diameter of lesions was measured and then compared with that on the untreated plot, whereby the effect was judged according to the same criterion as described in Test Example 1.

The results obtained are shown in Table 3.

Table 3

| No | Eg | Pc | Ps | Pi | Bc Curing |
|---|---|---|---|---|---|
| 2 | A | A | A | A | A |
| 3 | A | A | A | C | A |
| 4 | A | D | A | D | A |
| 5 | A | A | A | B | A |
| 6 | D | A | A | D | D |
| 7 | A | A | A | A | A |
| 8 | D | A | B | C | D |
| 9 | C | D | A | C | A |
| 10 | A | A | A | A | A |
| 11 | B | B | A | C | A |
| 12 | A | A | A | A | A |
| 13 | A | A | A | A | A |
| 14 | C | D | C | D | A |
| 15 | A | A | A | D | A |
| 16 | A | A | C | C | A |
| 17 | D | B | A | D | D |
| 18 | D | A | B | D | A |
| 20 | A | B | B | B | A |
| 21 | A | A | A | A | A |
| 22 | A | A | B | B | A |
| 23 | A | A | A | B | D |
| 24 | A | A | A | A | A |
| 25 | A | A | A | A | A |
| 26 | A | A | A | C | A |

- Cont'd -

Table 3 (Cont'd)

| No | Eg | Pc | Ps | Pi | Bc Curing |
|---|---|---|---|---|---|
| 27 | A | A | A | A | A |
| 28 | C | A | B | D | A |
| 29 | A | A | A | C | A |
| 30 | A | A | A | B | A |
| 31 | A | A | B | D | C |
| 32 | A | A | A | A | A |
| 33 | B | A | A | D | A |
| 34 | A | A | A | C | A |
| 35 | A | A | A | A | D |
| 36 | A | A | A | A | A |
| 37 | A | A | A | A | A |
| 38 | A | A | A | B | A |
| 39 | A | A | A | B | B |
| 40 | A | A | A | A | A |
| 41 | A | A | A | A | A |
| 42 | B | A | A | A | A |
| 43 | A | A | A | A | A |
| 44 | A | A | A | A | A |
| 45 | A | A | A | A | A |
| 46 | A | A | A | A | A |
| 47 | A | A | A | B | A |
| 48 | A | A | A | A | A |
| 49 | A | A | A | A | A |
| 50 | A | A | A | B | A |

- Cont'd -

Table 3 (Cont'd)

| No | Eg | Pc | Ps | Pi | Bc Curing |
|---|---|---|---|---|---|
| 51 | A | A | A | A | A |
| 52 | A | A | A | B | A |
| 53 | A | A | A | A | A |
| 54 | A | A | A | B | A |
| 55 | A | A | A | A | A |
| 56 | A | A | A | B | A |
| 57 | A | A | A | B | A |
| 58 | A | A | A | C | A |
| 59 | A | A | A | A | A |
| 60 | A | A | A | B | A |
| 61 | A | A | A | B | A |
| 62 | A | A | A | A | A |
| 63 | A | A | A | A | B |
| 64 | A | A | A | C | A |
| 65 | A | A | A | A | A |
| 66 | D | A | A | D | A |
| 67 | B | A | D | A | A |
| 68 | A | B | A | B | A |
| 69 | A | A | A | A | A |
| 70 | A | A | A | A | A |
| 71 | B | A | A | A | A |
| 72 | A | A | B | A | A |
| 73 | C | A | A | D | D |
| 74 | A | A | A | A | A |

- Cont'd -

Table 3 (Cont'd)

| No | Eg | Pc | Ps | Pi | Bc Curing |
|---|---|---|---|---|---|
| 75 | A | A | A | A | A |
| 76 | A | D | A | A | A |
| 77 | A | B | B | A | A |
| 78 | A | A | A | B | A |
| 79 | A | A | A | A | A |
| 80 | B | A | B | B | A |
| 81 | B | A | A | D | A |
| 82 | A | A | A | A | A |
| 83 | A | A | A | D | A |
| 84 | A | A | B | A | A |
| 85 | A | A | A | A | B |
| 86 | B | A | A | A | A |
| 87 | A | A | A | B | A |
| 88 | A | A | A | A | A |
| 89 | A | A | A | B | A |
| 90 | A | A | A | D | A |
| 91 | A | B | B | B | A |
| 92 | A | A | B | A | A |
| 93 | A | A | A | B | A |
| 94 | B | A | A | D | A |
| 95 | A | A | A | C | A |
| 96 | A | A | A | A | A |
| 97 | A | B | C | B | B |
| 99 | D | A | A | C | A |
| 100 | A | A | A | D | A |

- Cont'd -

Table 3 (Cont'd)

| No | Eg | Pc | Ps | Pi | Bc Curing |
|---|---|---|---|---|---|
| 101 | A | A | A | B | A |
| 102 | C | C | B | B | A |
| 103 | A | A | A | A | A |
| 104 | A | A | A | D | A |
| 105 | A | A | A | B | B |
| 106 | B | A | C | C | D |
| 107 | A | A | B | B | D |
| 108 | A | A | B | A | A |
| 109 | B | A | A | A | A |
| 110 | A | A | B | C | A |
| 111 | A | A | A | B | A |
| 112 | A | A | B | D | A |
| 113 | A | A | C | B | A |
| 114 | A | C | B | C | B |
| 115 | A | B | B | C | A |
| 116 | A | A | A | C | B |
| 117 | A | A | A | B | A |
| 118 | C | A | D | B | C |
| 119 | A | A | A | A | A |
| 120 | B | B | B | B | A |
| 121 | A | A | A | C | A |
| 122 | A | A | B | A | A |
| 123 | A | A | A | C | A |
| 124 | A | A | C | B | A |

- Cont'd -

Table 3 (Cont'd)

| No | Eg | Pc | Ps | Pi | Bc Curing |
|---|---|---|---|---|---|
| 125 | A | A | A | C | A |
| 127 | A | D | B | B | C |
| 128 | A | A | C | A | C |
| 129 | A | A | D | A | D |
| 130 | C | A | D | B | B |
| 131 | D | A | B | D | D |
| 133 | A | C | A | C | A |
| 134 | B | A | D | D | C |
| 135 | B | A | B | A | B |
| 136 | B | A | D | D | D |
| 138 | A | D | C | C | A |
| 139 | A | D | C | D | A |
| 140 | A | A | A | A | A |
| 141 | A | A | A | A | A |
| 142 | A | A | A | A | A |
| 143 | A | A | C | B | B |
| 144 | A | A | A | D | B |
| 145 | A | A | C | C | D |
| 146 | A | A | A | D | B |
| 147 | A | A | A | C | A |
| 148 | B | A | A | C | A |
| 149 | A | A | D | C | A |
| 150 | A | A | A | B | A |
| 151 | A | A | A | A | A |
| 152 | A | D | A | C | A |

- Cont'd -

Table 3 (Cont'd)

| No | Eg | Pc | Ps | Pi | Bc Curing |
|---|---|---|---|---|---|
| 153 | C | A | A | D | A |
| 154 | C | A | A | B | A |
| 155 | A | A | A | D | A |
| 156 | A | A | A | B | A |
| 157 | A | A | B | C | C |
| 158 | A | A | A | A | A |
| 159 | A | D | B | C | C |
| 160 | A | A | A | A | A |
| 161 | A | A | A | B | A |
| 162 | A | A | A | A | A |
| 163 | A | A | A | D | A |
| 165 | D | A | A | C | C |
| 166 | A | A | B | B | B |
| 169 | A | A | A | A | A |
| 168 | D | A | B | D | B |
| 170 | C | B | A | B | A |
| 176 | A | A | A | A | A |
| 177 | A | A | A | A | A |
| 178 | A | A | A | A | A |
| 179 | A | - | A | - | A |
| 180 | A | A | A | - | C |
| 181 | A | - | A | B | B |
| 182 | A | - | A | - | A |
| 183 | A | - | A | B | A |

- Cont'd -

Table 3 (Cont'd)

| No | Eg | Pc | Ps | Pi | Bc Curing |
|---|---|---|---|---|---|
| 184 | A | - | A | - | B |
| 185 | A | - | A | - | A |
| 186 | A | A | B | B | A |
| 187 | A | A | A | - | A |
| 188 | A | - | A | - | A |
| 189 | A | A | B | C | A |
| 193 | A | A | C | - | A |
| 194 | C | A | C | - | - |
| 196 | A | B | A | B | A |
| 197 | A | A | A | A | A |
| 198 | A | - | A | - | B |
| 199 | A | A | A | - | B |
| 200 | A | B | - | - | - |
| 201 | A | B | C | B | - |
| 202 | A | A | C | C | - |
| 203 | A | A | B | C | A |
| 204 | A | A | A | C | - |
| 205 | A | A | A | A | A |
| 206 | A | A | A | A | A |
| 207 | A | - | A | - | B |
| 208 | A | - | A | - | B |
| 209 | A | A | A | A | A |
| 210 | B | B | B | - | - |

- Cont'd -

Table 3 (Cont'd)

| No | Eg | Pc | Ps | Pi | Bc Curing |
|---|---|---|---|---|---|
| 211 | A | B | C | - | - |
| 212 | A | - | C | - | - |
| 213 | A | - | B | - | - |

Note: Eg barley powdery mildew
Pc cucumber downy mildew
Pc rice blast
Pi tomato late blight
Bc cucumber gray mold

## Claims

**1.** A benzylhydrazone derivative represented by the general formula (I):

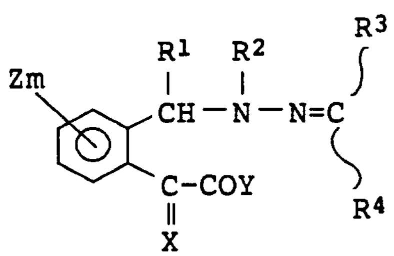

(I)

wherein X is a group represented by the formula:

CH~$OR^5$

(wherein $R^5$ is a $C_{1-6}$alkyl group) or a group represented by the formula:

N~$OR^5$

(wherein $R^5$ is as defined above), Y is a $C_{1-6}$alkoxy group, a $C_{1-6}$alkylthio group, an unsubstituted amino group, or a mono- or di-substituted amino group having as the substituent(s) one or two $C_{1-6}$alkyl groups which may be the same or different, Z's, which may be the same or different, are halogen atoms, $C_{1-6}$alkyl groups or halo-$C_{1-6}$alkyl groups, m is zero or an integer of 1 to 4, $R^1$ is a hydrogen atom, a $C_{1-6}$alkyl group or a halo-$C_{1-6}$alkyl group, $R^2$ is a formyl group; a $C_{1-6}$alkylcarbonyl

group; a halo-$C_{1-6}$alkylcarbonyl group; a $C_{1-6}$alkylsulfonyl group; a halo-$C_{1-6}$alkylsulfonyl group; an unsubstituted aminocarbonyl group; a mono- or di-substituted aminocarbonyl group having one or two substituents which may be the same or different and are selected from the group consisting of formyl group and $C_{1-6}$alkyl groups; a $C_{1-6}$alkoxycarbonyl group; a $C_{1-6}$alkoxycarbonyl-$C_{1-6}$alkylcarbonyl group; a cyano-$C_{1-6}$alkylcarbonyl group; a $C_{1-6}$alkylthiocarbonyl group; an unsubstituted phenylcarbonyl group; a substituted phenylcarbonyl group having 1 to 5 substituents which may be the same or different and are selected from the group consisting of halogen atoms, $C_{1-6}$alkyl groups, halo-$C_{1-6}$alkyl groups, $C_{1-6}$alkoxy groups and halo-$C_{1-6}$alkoxy groups; an unsubstituted phenyl-$C_{1-6}$alkylcarbonyl group; a substituted phenyl-$C_{1-6}$alkylcarbonyl group having on the phenyl ring 1 to 5 substituents which may be the same or different and are selected from the group consisting of halogen atoms, $C_{1-6}$alkyl groups, halo-$C_{1-6}$alkyl groups, $C_{1-6}$alkoxy groups and halo-$C_{1-6}$alkoxy groups; an unsubstituted phenoxycarbonyl group; a substituted phenoxycarbonyl group having on the phenyl ring 1 to 5 substituents which may be the same or different and are selected from the group consisting of halogen atoms, $C_{1-6}$alkyl groups, halo-$C_{1-6}$alkyl groups, $C_{1-6}$alkoxy groups and halo-$C_{1-6}$alkoxy groups; an unsubstituted benzyloxycarbonyl group; a substituted benzyloxycarbonyl group having on the phenyl ring 1 to 5 substituents which may be the same or different and are selected from the group consisting of halogen atoms, $C_{1-6}$alkyl groups, halo-$C_{1-6}$alkyl groups, $C_{1-6}$alkoxy groups and halo-$C_{1-6}$alkoxy groups; or a di-substituted phosphonyl group having as the substituents two $C_{1-6}$alkyl groups which may be the same or different, and $R^3$ and $R^4$, which may be the same or different, are hydrogen atoms; cyano groups; $C_{1-6}$alkyl groups; halo-$C_{1-6}$alkyl groups; $C_{3-6}$cycloalkyl groups; cyano-$C_{1-6}$alkyl groups; $C_{2-6}$alkenyl groups; $C_{3-6}$cycloalkenyl groups; $C_{1-6}$alkoxy groups; halo-$C_{1-6}$alkoxy groups; $C_{1-6}$alkylthio groups; halo-$C_{1-6}$alkylthio groups; $C_{1-6}$alkylsulfinyl groups; $C_{1-6}$alkylsulfonyl groups; $C_{2-6}$alkenylthio groups; $C_{2-6}$alkynylthio groups; $C_{1-6}$alkoxy-$C_{1-6}$alkyl groups; $C_{1-6}$alkoxy-$C_{1-6}$alkylthio groups; $C_{1-6}$alkylthio-$C_{1-6}$alkyl groups; $C_{1-6}$alkylsulfonyl-$C_{1-6}$alkyl groups; $C_{3-6}$cycloalkylthio groups; $C_{1-6}$alkoxycarbonyl-$C_{1-6}$alkylthio-$C_{1-6}$alkyl groups; $C_{1-6}$alkoxycarbonylthio groups; unsubstituted phenyl groups; substituted phenyl groups having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkylsulfinyl groups, $C_{1-6}$alkylsulfonyl groups, unsubstituted aminocarbonyl group, mono- or di-substituted aminocarbonyl groups having one or two substituents which may be the same or different and are selected from the group consisting of $C_{1-6}$alkyl groups and $C_{1-6}$alkylcarbonyl groups, cyano-$C_{1-6}$alkoxy groups, $C_{1-6}$alkoxycarbonyloxy groups, unsubstituted amino group, mono- or di-substituted amino groups having one or two substituents which may be the same or different and are selected from the group consisting of formyl group, $C_{1-6}$alkyl groups and $C_{1-6}$alkylcarbonyl groups, $C_{1-6}$alkoxycarbonyl groups, unsubstituted aminocarbonyloxy group, mono- or di-substituted aminocarbonyloxy groups having one or two substituents which may be the same or different and are selected from $C_{1-6}$alkyl groups, methylenedioxy group, unsubstituted phenyl group, substituted phenyl groups having 1 to 5 substituents which may be the same or different and are selected from the group consisting of halogen atoms, $C_{1-6}$alkyl groups and $C_{1-6}$alkoxy groups, unsubstituted phenoxy group, substituted phenoxy groups having 1 to 5 substituents which may be the same or different and are selected from the group consisting of halogen atoms, cyano group, nitro group, $C_{1-6}$alkyl groups and halo-$C_{1-6}$alkyl groups, unsubstituted phenyl-$C_{1-6}$alkyl groups, substituted phenyl-$C_{1-6}$alkyl groups having on the phenyl ring 1 to 5 substituents which may be the same or different and are selected from the group consisting of halogen atoms, cyano group, nitro group, $C_{1-6}$alkyl groups and halo-$C_{1-6}$alkyl groups, unsubstituted phenyl-$C_{1-6}$alkoxy groups, substituted phenyl-$C_{1-6}$alkoxy groups having on the phenyl ring 1 to 5 substituents which may be the same or different and are selected from the group consisting of halogen atoms, cyano group, nitro group, $C_{1-6}$alkyl groups and halo-$C_{1-6}$alkyl groups, unsubstituted morpholino group, substituted morpholino groups having as the substituent(s) one or more $C_{1-6}$alkyl groups which may be the same or different, unsubstituted pyrimidinyloxy group, substituted pyrimidinyloxy groups having as the substituent(s) one or more $C_{1-6}$alkyl groups which may be the same or different, unsubstituted morpholinocarbonyl group, substituted morpholinocarbonyl groups having as the substituent(s) one or more $C_{1-6}$alkyl groups which may be the same or different, and $C_{3-4}$alkylene groups which each form a bicyclic ring together with the adjacent carbon atom of the phenyl ring; unsubstituted phenyl-$C_{1-6}$alkylthio groups; substituted phenyl-$C_{1-6}$alkylthio groups having on the phenyl ring 1 to 5 substituents which may be the same or different and are selected from the group consisting of $C_{1-6}$alkyl groups and halo-$C_{1-6}$alkyl groups; unsubstituted phenylsulfonyl groups; substituted phenylsulfonyl groups having on the phenyl ring 1 to 5 substituents which may be the same or different and

are selected from the group consisting of $C_{1-6}$alkyl groups and halo-$C_{1-6}$alkyl groups; unsubstituted phenylsulfonyl-$C_{1-6}$alkyl groups; substituted phenylsulfonyl-$C_{1-6}$alkyl groups having on the phenyl ring 1 to 5 substituents which may be the same or different and are selected from the group consisting of $C_{1-6}$alkyl groups and halo-$C_{1-6}$alkyl groups; unsubstituted phenylthio-$C_{1-6}$alkyl groups; substituted phenylthio-$C_{1-6}$alkyl groups having on the phenyl ring 1 to 5 substituents which may be the same or different and are selected from the group consisting of $C_{1-6}$alkyl groups and halo-$C_{1-6}$alkyl groups; unsubstituted phenyl-$C_{1-6}$alkylthio-$C_{1-6}$alkyl groups; substituted phenyl-$C_{1-6}$alkylthio-$C_{1-6}$alkyl groups having on the phenyl ring 1 to 5 substituents which may be the same or different and are selected from the group consisting of $C_{1-6}$alkyl groups and halo-$C_{1-6}$alkyl groups; unsubstituted amino-$C_{1-6}$alkyl groups; mono- or di-substituted amino-$C_{1-6}$alkyl groups having one or two substituents which may be the same or different and are selected from the group consisting of $C_{1-6}$alkyl groups, halo-$C_{1-6}$alkyl groups, $C_{1-6}$alkylcarbonyl groups, unsubstituted phenylcarbonyl group, substituted phenylcarbonyl groups having on the phenyl ring 1 to 5 substituents which may be the same or different and are selected from the group consisting of $C_{1-6}$alkyl groups and halo-$C_{1-6}$alkyl groups, phenyl group and benzyl group; furfurylthio groups; furfurylthio-$C_{1-6}$alkyl groups; unsubstituted morpholino-$C_{1-6}$alkyl groups; substituted morpholino-$C_{1-6}$alkyl groups having as the substituent(s) one or more $C_{1-6}$alkyl groups which may be the same or different; groups represented by the formula:

-CO-R6

(wherein R6 is a $C_{1-6}$alkyl group; a halo-$C_{1-6}$alkyl group; a $C_{1-6}$alkoxy group; a halo-$C_{1-6}$alkoxy group; a $C_{1-6}$alkylthio group; a halo-$C_{1-6}$alkylthio group; an unsubstituted amino group; a mono- or di-substituted amino group having one or two substituents which may be the same or different and are selected from the group consisting of $C_{1-6}$alkyl groups, halo-$C_{1-6}$alkyl groups and phenyl group; an unsubstituted phenyl group; a substituted phenyl group having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, unsubstituted amino group, mono- or di-substituted amino groups having one or two substituents which may be the same or different and are selected from the group consisting of $C_{1-6}$alkyl groups and $C_{1-6}$alkylcarbonyl groups, unsubstituted phenyl group, substituted phenyl groups having 1 to 5 substituents which may be the same or different and are selected from the group consisting of halogen atoms, $C_{1-6}$alkyl groups and halo-$C_{1-6}$alkyl groups, unsubstituted phenoxy group, substituted phenoxy groups having 1 to 5 substituents which may be the same or different and are selected from the group consisting of halogen atoms, cyano group, nitro group, $C_{1-6}$alkyl groups and halo-$C_{1-6}$alkyl groups, benzyl group, unsubstituted benzyloxy group, substituted benzyloxy groups having on the phenyl ring 1 to 5 substituents which may be the same or different and are selected from the group consisting of halogen atoms, $C_{1-6}$alkyl groups and halo-$C_{1-6}$alkyl groups, unsubstituted pyridyloxy group, substituted pyridyloxy groups having 1 to 4 substituents which may be the same or different and are selected from the group consisting of halogen atoms, $C_{1-6}$alkyl groups and halo-$C_{1-6}$alkyl groups, methylenedioxy group, and $C_{3-4}$alkylene groups which each form a bicyclic ring together with the adjacent carbon atom of the phenyl ring; an unsubstituted benzyloxy group; a substituted benzyloxy group having on the phenyl ring 1 to 5 substituents which may be the same or different and are selected from the group consisting of halogen atoms, $C_{1-6}$alkyl groups, halo-$C_{1-6}$alkyl groups, $C_{1-6}$alkoxy groups, halo-$C_{1-6}$alkoxy groups, $C_{1-6}$alkylthio groups and halo-$C_{1-6}$alkylthio groups; or a 5- or 6-membered heterocyclic ring having 1 to 3 heteroatoms which may be the same or different and are selected from the group consisting of oxygen atom, sulfur atom and nitrogen atom, said heterocyclic ring being able to have one or more substituents selected from the group consisting of $C_{1-6}$alkyl groups, halo-$C_{1-6}$alkyl groups, $C_{1-6}$alkoxy groups and halo-$C_{1-6}$alkoxy groups); unsubstituted amino groups; mono- or di-substituted amino groups having one or two substituents which may be the same or different and are selected from the group consisting of $C_{1-6}$alkyl groups, halo-$C_{1-6}$alkyl groups, $C_{2-6}$alkenyl groups, $C_{2-6}$alkynyl groups, $C_{1-6}$alkoxy-$C_{1-6}$alkyl groups, di-$C_{1-6}$alkoxy-$C_{1-6}$alkyl groups, amino-$C_{1-6}$alkyl groups, substituted mono- or di-$C_{1-6}$alkylamino-$C_{1-6}$alkyl groups having as the substituent(s) one or more $C_{1-6}$alkyl groups which may be the same or different, unsubstituted phenyl group, substituted phenyl groups having 1 to 5 substituents which may be the same or different and are selected from the group consisting of halogen atoms, $C_{1-6}$alkyl groups, $C_{1-6}$alkoxy groups, $C_{1-6}$alkylthio groups and halo-$C_{1-6}$alkyl groups, pyridyl-$C_{1-6}$alkyl groups, and morpholino-$C_{1-6}$alkyl groups; unsubstituted phenyl-$C_{1-6}$alkyl groups; substituted phenyl-$C_{1-6}$alkyl groups having on the phenyl ring 1 to 5 substituents which may be the same or different and are

selected from the group consisting of halogen atoms, $C_{1-6}$alkyl groups, halo-$C_{1-6}$alkyl groups, $C_{1-6}$alkoxy groups, halo-$C_{1-6}$alkoxy groups, $C_{1-6}$alkylthio groups and halo-$C_{1-6}$alkylthio groups; unsubstituted phenyl-$C_{2-6}$alkenyl groups; substituted phenyl-$C_{2-6}$alkenyl groups having on the phenyl ring 1 to 5 substituents which may be the same or different and are selected from the group consisting of halogen atoms, $C_{1-6}$alkyl groups and halo-$C_{1-6}$alkyl groups; naphthyl groups; 5- to 7-membered heterocyclic rings having 1 to 3 heteroatoms which may be the same or different and are selected from the group consisting of oxygen atom, sulfur atom and nitrogen atom; or 5- to 7-membered heterocyclic ring fused with a benzene ring or a $C_{4-6}$cycloalkane ring; the above heterocyclic rings being able to have one or more substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxycarbonyl groups, unsubstituted phenyl group, substituted phenyl groups having 1 to 5 substituents which may be the same or different and are selected from halogen atoms, benzyl group, pyridyl group, pyrimidyl group and dioxolane group, $R^3$ and $R^4$ being able to be taken together to represent a $C_{1-6}$-alkylene group or a $C_{2-6}$-alkenylene group, which may contain between adjacent carbon atoms of the carbon chain an oxygen atom, a sulfur atom, a nitrogen atom, a carbonyl group, or a nitrogen atom having a $C_{1-6}$alkyl or phenyl group bonded thereto, said alkylene or alkenylene group being able to have on the carbon chain one or more substituents which may be the same or different and are selected from the group consisting of $C_{1-6}$alkyl groups, halo-$C_{1-6}$alkyl groups, $C_{1-6}$alkoxy groups and halo-$C_{1-6}$alkoxy groups, and to form fused rings with a benzene ring.

**2.** A benzylhydrazone derivative according to claim 1, wherein X is a group represented by the formula:

CH~$OR^5$

(wherein $R^5$ is a $C_{1-6}$alkyl group) or a group represented by the formula:

N~$OR^5$

(wherein $R^5$ is as defined above), Y is a $C_{1-6}$alkoxy group, a $C_{1-6}$alkylthio group, an unsubstituted amino group, or a mono- or di-substituted amino group having as the substituent(s) one or two $C_{1-6}$alkyl groups which may be the same or different, Z's, which may be the same or different, are halogen atoms, $C_{1-6}$alkyl groups or halo-$C_{1-6}$alkyl groups, m is zero or an integer of 1 to 4, $R^1$ is a hydrogen atom, a $C_{1-6}$alkyl group or a halo-$C_{1-6}$alkyl group, $R^2$ is a $C_{1-6}$alkylcarbonyl group; a halo-$C_{1-6}$alkylcarbonyl group; a $C_{1-6}$alkylsulfonyl group; a halo-$C_{1-6}$alkylsulfonyl group; an unsubstituted aminocarbonyl group; a mono- or di-substituted aminocarbonyl group having one or two substituents which may be the same or different and are selected from the group consisting of formyl group and $C_{1-6}$alkyl groups; a $C_{1-6}$alkoxycarbonyl group; a $C_{1-6}$alkoxycarbonyl-$C_{1-6}$alkylcarbonyl group; a cyano-$C_{1-6}$alkylcarbonyl group; a $C_{1-6}$alkylthiocarbonyl group; an unsubstituted phenylcarbonyl group; a substituted phenylcarbonyl group having 1 to 5 substituents which may be the same or different and are selected from the group consisting of halogen atoms, $C_{1-6}$alkyl groups, halo-$C_{1-6}$alkyl groups, $C_{1-6}$alkoxy groups and halo-$C_{1-6}$alkoxy groups; an unsubstituted phenyl-$C_{1-6}$alkylcarbonyl group; a substituted phenyl-$C_{1-6}$alkylcarbonyl group having on the phenyl ring 1 to 5 substituents which may be the same or different and are selected from the group consisting of halogen atoms, $C_{1-6}$alkyl groups, halo-$C_{1-6}$alkyl groups, $C_{1-6}$alkoxy groups and halo-$C_{1-6}$alkoxy groups; an unsubstituted phenoxycarbonyl group; a substituted phenoxycarbonyl group having on the phenyl ring 1 to 5 substituents which may be the same or different and are selected from the group consisting of halogen atoms, $C_{1-6}$alkyl groups, halo-$C_{1-6}$alkyl groups, $C_{1-6}$alkoxy groups and halo-$C_{1-6}$alkoxy groups; an unsubstituted benzyloxycarbonyl group; a substituted benzyloxycarbonyl group having on the phenyl ring 1 to 5 substituents which may be the same or different and are selected from the group consisting of halogen atoms, $C_{1-6}$alkyl groups, halo-$C_{1-6}$alkyl groups, $C_{1-6}$alkoxy groups and halo-$C_{1-6}$alkoxy groups; or a di-substitued phosphonyl group having as the substituents two $C_{1-6}$alkyl groups which may be the same or different, and $R^3$ and $R^4$, which may be the same or different, are hydrogen atoms; cyano groups; $C_{1-6}$alkyl groups; halo-$C_{1-6}$alkyl groups; $C_{3-6}$cycloalkyl groups; cyano-$C_{1-6}$alkyl groups; $C_{2-6}$alkenyl groups; $C_{3-6}$cycloalkenyl groups; $C_{1-6}$alkoxy groups; halo-$C_{1-6}$alkoxy groups; $C_{1-6}$alkylthio groups; halo-$C_{1-6}$alkylthio groups; $C_{1-6}$alkylsulfinyl groups; $C_{1-6}$alkylsulfonyl groups; $C_{2-6}$alkenylthio groups; $C_{2-6}$alkynylthio groups; $C_{1-6}$alkoxy-$C_{1-6}$alkyl groups; $C_{1-6}$alkoxy-$C_{1-6}$alkylthio groups; $C_{1-6}$alkylthio-$C_{1-6}$alkyl groups; $C_{1-6}$alkylsulfonyl-$C_{1-6}$alkyl groups; $C_{3-6}$cycloalkylthio groups; $C_{1-6}$al-

koxycarbonyl-$C_{1-6}$alkylthio-$C_{1-6}$alkyl groups; $C_{1-6}$alkoxycarbonylthio groups; unsubstituted phenyl groups; substituted phenyl groups having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkylsulfinyl groups, $C_{1-6}$alkylsulfonyl groups, unsubstituted aminocarbonyl group, mono- or di-substituted aminocarbonyl groups having one or two substituents which may be the same or different and are selected from the group consisting of $C_{1-6}$alkyl groups and $C_{1-6}$alkylcarbonyl groups, cyano-$C_{1-6}$alkoxy groups, $C_{1-6}$alkoxycarbonyloxy groups, unsubstituted amino group, mono- or di-substituted amino groups having one or two substituents which may be the same or different and are selected from the group consisting of formyl group, $C_{1-6}$alkyl groups and $C_{1-6}$alkylcarbonyl groups, $C_{1-6}$alkoxycarbonyl groups, unsubstituted aminocarbonyloxy group, mono- or di-substituted aminocarbonyloxy groups having one or two substituents which may be the same or different and are selected from $C_{1-6}$alkyl groups, methylenedioxy group, unsubstituted phenyl group, substituted phenyl groups having 1 to 5 substituents which may be the same or different and are selected from the group consisting of halogen atoms, $C_{1-6}$alkyl groups and $C_{1-6}$alkoxy groups, unsubstituted phenoxy group, substituted phenoxy groups having 1 to 5 substituents which may be the same or different and are selected from the group consisting of halogen atoms, cyano group, nitro group, $C_{1-6}$alkyl groups and halo-$C_{1-6}$alkyl groups, unsubstituted phenyl-$C_{1-6}$alkyl groups, substituted phenyl-$C_{1-6}$alkyl groups having on the phenyl ring 1 to 5 substituents which may be the same or different and are selected from the group consisting of halogen atoms, cyano group, nitro group, $C_{1-6}$alkyl groups and halo-$C_{1-6}$alkyl groups, unsubstituted phenyl-$C_{1-6}$alkoxy groups, substituted phenyl-$C_{1-6}$alkoxy groups having on the phenyl ring 1 to 5 substituents which may be the same or different and are selected from the group consisting of halogen atoms, cyano group, nitro group, $C_{1-6}$alkyl groups and halo-$C_{1-6}$alkyl groups, unsubstituted morpholino group, substituted morpholino groups having as the substituent(s) one or more $C_{1-6}$alkyl groups which may be the same or different, unsubstituted pyrimidinyloxy group, substituted pyrimidinyloxy groups having as the substituent(s) one or more $C_{1-6}$alkyl groups which may be the same or different, unsubstituted morpholinocarbonyl group, substituted morpholinocarbonyl groups having as the substituent(s) one or more $C_{1-6}$alkyl groups which may be the same or different, and $C_{3-4}$alkylene groups which each form a bicyclic ring together with the adjacent carbon atoms of the phenyl ring; unsubstituted phenyl-$C_{1-6}$alkylthio groups; substituted phenyl-$C_{1-6}$alkylthio groups having on the phenyl ring 1 to 5 substituents which may be the same or different and are selected from the group consisting of $C_{1-6}$alkyl groups and halo-$C_{1-6}$alkyl groups; unsubstituted phenylsulfonyl groups; substituted phenylsulfonyl groups having on the phenyl ring 1 to 5 substituents which may be the same or different and are selected from the group consisting of $C_{1-6}$alkyl groups and halo-$C_{1-6}$alkyl groups; unsubstituted phenylsulfonyl-$C_{1-6}$alkyl groups; substituted phenylsulfonyl-$C_{1-6}$alkyl groups having on the phenyl ring 1 to 5 substituents which may be the same or different and are selected from the group consisting of $C_{1-6}$alkyl groups and halo-$C_{1-6}$alkyl groups; unsubstituted phenylthio-$C_{1-6}$alkyl groups; substituted phenylthio-$C_{1-6}$alkyl groups having on the phenyl ring 1 to 5 substituents which may be the same or different and are selected from the group consisting of $C_{1-6}$alkyl groups and halo-$C_{1-6}$alkyl groups; unsubstituted phenyl-$C_{1-6}$alkylthio-$C_{1-6}$alkyl groups; substituted phenyl-$C_{1-6}$alkylthio-$C_{1-6}$alkyl groups having on the phenyl ring 1 to 5 substituents which may be the same or different and are selected from the group consisting of $C_{1-6}$alkyl groups and halo-$C_{1-6}$alkyl groups; unsubstituted amino-$C_{1-6}$alkyl groups; mono- or di-substituted amino-$C_{1-6}$alkyl groups having one or two substituents which may be the same or different and are selected from the group consisting of $C_{1-6}$alkyl groups, halo-$C_{1-6}$alkyl groups, $C_{1-6}$alkylcarbonyl groups, unsubstituted phenylcarbonyl group, substituted phenylcarbonyl groups having on the phenyl ring 1 to 5 substituents which may be the same or different and are selected from the group consisting of $C_{1-6}$alkyl groups and halo-$C_{1-6}$alkyl groups, phenyl group and benzyl group; furfurylthio groups; furfurylthio-$C_{1-6}$alkyl groups; unsubstituted morpholino-$C_{1-6}$alkyl groups; substituted morpholino-$C_{1-6}$alkyl groups having as the substituent(s) one or more $C_{1-6}$alkyl groups which may be the same or different; unsubstituted amino groups; mono- or di-substituted amino groups having one or two substituents which may be the same or different and are selected from the group consisting of $C_{1-6}$alkyl groups, halo-$C_{1-6}$alkyl groups, $C_{2-6}$alkenyl groups, $C_{2-6}$alkynyl groups, $C_{1-6}$alkoxy-$C_{1-6}$alkyl groups, di-$C_{1-6}$alkoxy-$C_{1-6}$alkyl groups, amino-$C_{1-6}$alkyl groups, substituted mono- or di-$C_{1-6}$alkylamino-$C_{1-6}$alkyl groups having as the substituent(s) one or more $C_{1-6}$alkyl groups which may be the same or different, unsubstituted phenyl group, substituted phenyl groups having 1 to 5 substituents which may be the same or different and are selected from the group consisting of halogen atoms, $C_{1-6}$alkyl groups, $C_{1-6}$alkoxy groups, $C_{1-6}$alkylthio groups and halo-$C_{1-6}$alkyl groups, pyridyl-$C_{1-6}$alkyl groups, and morpholino-$C_{1-6}$alkyl

groups; unsubstituted phenyl-$C_{1-6}$alkyl groups; substituted phenyl-$C_{1-6}$alkyl groups having on the phenyl ring 1 to 5 substituents which may be the same or different and are selected from the group consisting of halogen atoms, $C_{1-6}$alkyl groups, halo-$C_{1-6}$alkyl groups, $C_{1-6}$alkoxy groups, halo-$C_{1-6}$alkoxy groups, $C_{1-6}$alkylthio groups and halo-$C_{1-6}$alkylthio groups; unsubstituted phenyl-$C_{2-6}$alkenyl groups; substituted phenyl-$C_{2-6}$alkenyl groups having on the phenyl ring 1 to 5 substituents which may be the same or different and are selected from the group consisting of halogen atoms, $C_{1-6}$alkyl groups and halo-$C_{1-6}$alkyl groups; naphthyl groups; 5- to 7-membered heterocyclic rings having 1 to 3 heteroatoms which may be the same or different and are selected from the group consisting of oxygen atom, sulfur atom and nitrogen atom; or 5- to 7-membered heterocyclic ring fused with a benzene ring or a $C_{4-6}$cycloalkane ring; the above heterocyclic rings being able to have one or more substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxycarbonyl groups, unsubstituted phenyl group, substituted phenyl groups having 1 to 5 substituents which may be the same or different and are selected from halogen atoms, benzyl group, pyridyl group, pyrimidyl group and dioxolane group, $R^3$ and $R^4$ being able to be taken together to represent a $C_{1-6}$-alkylene group or a $C_{2-6}$-alkenylene group, which may contain between adjacent carbon atoms of the carbon chain an oxygen atom, a sulfur atom, a nitrogen atom, a carbonyl group, or a nitrogen atom having a $C_{1-6}$alkyl or phenyl group bonded thereto, said alkylene or alkenylene group being able to have on the carbon chain one or more substituents which may be the same or different and are selected from the group consisting of $C_{1-6}$alkyl groups, halo-$C_{1-6}$alkyl groups, $C_{1-6}$alkoxy groups and halo-$C_{1-6}$alkoxy groups, and to form fused rings with a benzene ring.

**3.** A benzylhydrazone derivative according to claim 2, wherein X is a group represented by the formula:

CH~$OR^5$

(wherein $R^5$ is a $C_{1-6}$alkyl group) or a group represented by the formula:

N~$OR^5$

(wherein $R^5$ is as defined above), Y is a $C_{1-6}$alkoxy group, an unsubstituted amino group, or a mono- or di-substituted amino group having as the substituent(s) one or two $C_{1-6}$alkyl groups which may be the same or different, Z's, which may be the same or different, are halogen atoms, $C_{1-6}$alkyl groups or halo-$C_{1-6}$alkyl groups, m is zero, $R^1$ is a hydrogen atom, $R^2$ is a $C_{1-6}$alkylcarbonyl group; and $R^3$ and $R^4$, which may be the same or different, are $C_{1-6}$alkyl groups; $C_{1-6}$alkylthio groups; unsubstituted phenyl groups; substituted phenyl groups having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkylsulfinyl groups, $C_{1-6}$alkylsulfonyl groups and methylenedioxy group; 5- to 7-membered heterocyclic rings having 1 to 3 heteroatoms which may be the same or different and are selected from the group consisting of oxygen atom, sulfur atom and nitrogen atom; or 5- to 7-membered heterocyclic ring fused with a benzene ring or a $C_{4-6}$cycloalkane ring; the above heterocyclic rings being able to have one or more substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxycarbonyl groups, unsubstituted phenyl group, substituted phenyl groups having 1 to 5 substituents which may be the same or different and are selected from halogen atoms, benzyl group, pyridyl group, pyrimidyl group and dioxolane group.

**4.** A benzylhydrazone derivative according to claim 3, wherein the 5- to 7-membered heterocyclic ring being formed by $R^3$ and $R^4$ and having 1 to 3 heteroatoms which may be the same or different and are selected from the group consisting of oxygen atom, sulfur atom and nitrogen atom, or 5- to 7-membered heterocyclic ring fused with a benzene ring or a $C_{4-6}$cycloalkane ring, is furan, thiophene, pyrrole, pyrazole, imidazole, triazole, oxazole, thiazole, pyridine, pyridazine, pyrimidine, pyrazine, pyrrolidine, piperidine, morpholine, thiomorpholine, piperazine, hexamethyleneimine, tetrahydroquinoline, tetrahydroisoquinoline, 2,3-dihydro-1,4-benzoxazine, octahydro-1,4-benzoxazine or indole.

**5.** A process for producing a benzylhydrazone derivative represented by the general formula (I):

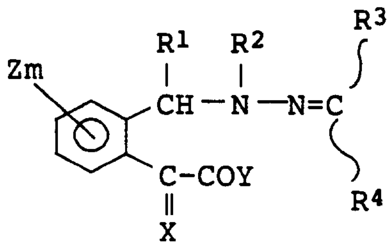

(I)

[wherein X is a group represented by the formula:

CH~OR$^5$

(wherein R$^5$ is a $C_{1-6}$alkyl group) or a group represented by the formula:

N~OR$^5$

(wherein R$^5$ is as defined above), Y is a $C_{1-6}$alkoxy group, a $C_{1-6}$alkylthio group, an unsubstituted amino group, or a mono- or di-substituted amino group having as the substituent(s) one or two $C_{1-6}$alkyl groups which may be the same or different, Z's, which may be the same or different, are halogen atoms, $C_{1-6}$alkyl groups or halo-$C_{1-6}$alkyl groups, m is zero or an integer of 1 to 4, R$^1$ is a hydrogen atom, a $C_{1-6}$alkyl group or a halo-$C_{1-6}$alkyl group, R$^2$ is a formyl group; a $C_{1-6}$alkylcarbonyl group; a halo-$C_{1-6}$alkylcarbonyl group; a $C_{1-6}$alkylsulfonyl group; a halo-$C_{1-6}$alkylsulfonyl group; an unsubstituted aminocarbonyl group; a mono- or di-substituted aminocarbonyl group having one or two substituents which may be the same or different and are selected from the group consisting of formyl group and $C_{1-6}$alkyl groups; a $C_{1-6}$alkoxycarbonyl group; a $C_{1-6}$alkoxycarbonyl-$C_{1-6}$alkylcarbonyl group; a cyano-$C_{1-6}$alkylcarbonyl group; a $C_{1-6}$alkylthiocarbonyl group; an unsubstituted phenylcarbonyl group; a substituted phenylcarbonyl group having 1 to 5 substituents which may be the same or different and are selected from the group consisting of halogen atoms, $C_{1-6}$alkyl groups, halo-$C_{1-6}$alkyl groups, $C_{1-6}$alkoxy groups and halo-$C_{1-6}$alkoxy groups; an unsubstituted phenyl-$C_{1-6}$alkylcarbonyl group; a substituted phenyl-$C_{1-6}$alkylcarbonyl group having on the phenyl ring 1 to 5 substituents which may be the same or different and are selected from the group consisting of halogen atoms, $C_{1-6}$alkyl groups, halo-$C_{1-6}$alkyl groups, $C_{1-6}$alkoxy groups and halo-$C_{1-6}$alkoxy groups; an unsubstituted phenoxycarbonyl group; a substituted phenoxycarbonyl group having on the phenyl ring 1 to 5 substituents which may be the same or different and are selected from the group consisting of halogen atoms, $C_{1-6}$alkyl groups, halo-$C_{1-6}$alkyl groups, $C_{1-6}$alkoxy groups and halo-$C_{1-6}$alkoxy groups; an unsubstituted benzyloxycarbonyl group; a substituted benzyloxycarbonyl group having on the phenyl ring 1 to 5 substituents which may be the same or different and are selected from the group consisting of halogen atoms, $C_{1-6}$alkyl groups, halo-$C_{1-6}$alkyl groups, $C_{1-6}$alkoxy groups and halo-$C_{1-6}$alkoxy groups; or a di-substituted phosphonyl group having as the substituents two $C_{1-6}$alkyl groups which may be the same or different, and R$^3$ and R$^4$, which may be the same or different, are hydrogen atoms; cyano groups; $C_{1-6}$alkyl groups; halo-$C_{1-6}$alkyl groups; $C_{3-6}$cycloalkyl groups; cyano-$C_{1-6}$alkyl groups; $C_{2-6}$alkenyl groups; $C_{3-6}$cycloalkenyl groups; $C_{1-6}$alkoxy groups; halo-$C_{1-6}$alkoxy groups; $C_{1-6}$alkylthio groups; halo-$C_{1-6}$alkylthio groups; $C_{1-6}$alkylsulfinyl groups; $C_{1-6}$alkylsulfonyl groups; $C_{2-6}$alkenylthio groups; $C_{2-6}$alkynylthio groups; $C_{1-6}$alkoxy-$C_{1-6}$alkyl groups; $C_{1-6}$alkoxy-$C_{1-6}$alkylthio groups; $C_{1-6}$alkylthio-$C_{1-6}$alkyl groups; $C_{1-6}$alkylsulfonyl-$C_{1-6}$alkyl groups; $C_{3-6}$cycloalkylthio groups; $C_{1-6}$alkoxycarbonyl-$C_{1-6}$alkylthio-$C_{1-6}$alkyl groups; $C_{1-6}$alkoxycarbonylthio groups; unsubstituted phenyl groups; substituted phenyl groups having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkylsulfinyl groups, $C_{1-6}$alkylsulfonyl groups, unsubstituted aminocarbonyl group, mono- or di-substituted aminocarbonyl groups having one or two substituents which may be the same or different and are selected from the group consisting of $C_{1-6}$alkyl groups and $C_{1-6}$alkylcarbonyl groups, cyano-$C_{1-6}$alkoxy groups, $C_{1-6}$alkoxycarbonyloxy

groups, unsubstituted amino group, mono- or di-substituted amino groups having one or two substituents which may be the same or different and are selected from the group consisting of formyl group, $C_{1-6}$alkyl groups and $C_{1-6}$alkylcarbonyl groups, $C_{1-6}$alkoxycarbonyl groups, unsubstituted aminocarbonyloxy group, mono- or di-substituted aminocarbonyloxy groups having one or two substituents which may be the same or different and are selected from $C_{1-6}$alkyl groups, methylenedioxy group, unsubstituted phenyl group, substituted phenyl groups having 1 to 5 substituents which may be the same or different and are selected from the group consisting of halogen atoms, $C_{1-6}$alkyl groups and $C_{1-6}$alkoxy groups, unsubstituted phenoxy group, substituted phenoxy groups having 1 to 5 substituents which may be the same or different and are selected from the group consisting of halogen atoms, cyano group, nitro group, $C_{1-6}$alkyl groups and halo-$C_{1-6}$alkyl groups, unsubstituted phenyl-$C_{1-6}$alkyl groups, substituted phenyl-$C_{1-6}$alkyl groups having on the phenyl ring 1 to 5 substituents which may be the same or different and are selected from the group consisting of halogen atoms, cyano group, nitro group, $C_{1-6}$alkyl groups and halo-$C_{1-6}$alkyl groups, unsubstituted phenyl-$C_{1-6}$alkoxy groups, substituted phenyl-$C_{1-6}$alkoxy groups having on the phenyl ring 1 to 5 substituents which may be the same or different and are selected from the group consisting of halogen atoms, cyano group, nitro group, $C_{1-6}$alkyl groups and halo-$C_{1-6}$alkyl groups, unsubstituted morpholino group, substituted morpholino groups having as the substituent(s) one or more $C_{1-6}$alkyl groups which may be the same or different, unsubstituted pyrimidinyloxy group, substituted pyrimidinyloxy groups having as the substituent(s) one or more $C_{1-6}$alkyl groups which may be the same or different, unsubstituted morpholinocarbonyl group, substituted morpholinocarbonyl groups having as the substituent(s) one or more $C_{1-6}$alkyl groups which may be the same or different, and $C_{3-4}$alkylene groups which each form a bicyclic ring together with the adjacent carbon atoms of the phenyl ring; unsubstituted phenyl-$C_{1-6}$alkylthio groups; substituted phenyl-$C_{1-6}$alkylthio groups having on the phenyl ring 1 to 5 substituents which may be the same or different and are selected from the group consisting of $C_{1-6}$alkyl groups and halo-$C_{1-6}$alkyl groups; unsubstituted phenylsulfonyl groups; substituted phenylsulfonyl groups having on the phenyl ring 1 to 5 substituents which may be the same or different and are selected from the group consisting of $C_{1-6}$alkyl groups and halo-$C_{1-6}$alkyl groups; unsubstituted phenylsulfonyl-$C_{1-6}$alkyl groups; substituted phenylsulfonyl-$C_{1-6}$alkyl groups having on the phenyl ring 1 to 5 substituents which may be the same or different and are selected from the group consisting of $C_{1-6}$alkyl groups and halo-$C_{1-6}$alkyl groups; unsubstituted phenylthio-$C_{1-6}$alkyl groups; substituted phenylthio-$C_{1-6}$alkyl groups having on the phenyl ring 1 to 5 substituents which may be the same or different and are selected from the group consisting of $C_{1-6}$alkyl groups and halo-$C_{1-6}$alkyl groups; unsubstituted phenyl-$C_{1-6}$alkylthio-$C_{1-6}$alkyl groups; substituted phenyl-$C_{1-6}$alkylthio-$C_{1-6}$alkyl groups having on the phenyl ring 1 to 5 substituents which may be the same or different and are selected from the group consisting of $C_{1-6}$alkyl groups and halo-$C_{1-6}$alkyl groups; unsubstituted amino-$C_{1-6}$alkyl groups; mono- or di-substituted amino-$C_{1-6}$alkyl groups having one or two substituents which may be the same or different and are selected from the group consisting of $C_{1-6}$alkyl groups, halo-$C_{1-6}$alkyl groups, $C_{1-6}$alkylcarbonyl groups, unsubstituted phenylcarbonyl group, substituted phenylcarbonyl groups having on the phenyl ring 1 to 5 substituents which may be the same or different and are selected from the group consisting of $C_{1-6}$alkyl groups and halo-$C_{1-6}$alkyl groups, phenyl group and benzyl group; furfurylthio groups; furfurylthio-$C_{1-6}$alkyl groups; unsubstituted morpholino-$C_{1-6}$alkyl groups; substituted morpholino-$C_{1-6}$alkyl groups having as the substituent(s) one or more $C_{1-6}$alkyl groups which may be the same or different; groups represented by the formula:

$-CO-R^6$

(wherein R6 is a $C_{1-6}$alkyl group; a halo-$C_{1-6}$alkyl group; a $C_{1-6}$alkoxy group; a halo-$C_{1-6}$alkoxy group; a $C_{1-6}$alkylthio group; a halo-$C_{1-6}$alkylthio group; an unsubstituted amino group; a mono- or di-substituted amino group having one or two substituents which may be the same or different and are selected from the group consisting of $C_{1-6}$alkyl groups, halo-$C_{1-6}$alkyl groups and phenyl group; an unsubstituted phenyl group; a substituted phenyl group having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, unsubstituted amino group, mono- or di-substituted amino groups having one or two substituents which may be the same or different and are selected from the group consisting of $C_{1-6}$alkyl groups and $C_{1-6}$alkylcarbonyl groups, unsubstituted phenyl group, substituted phenyl groups having 1 to 5 substituents which may be the same or different and are selected from the group consisting of halogen atoms, $C_{1-6}$alkyl groups and halo-$C_{1-6}$alkyl groups, unsubstituted phenoxy group, substituted phenoxy

groups having 1 to 5 substituents which may be the same or different and are selected from the group consisting of halogen atoms, cyano group, nitro group, $C_{1-6}$alkyl groups and halo-$C_{1-6}$alkyl groups, benzyl group, unsubstituted benzyloxy group, substituted benzyloxy groups having on the phenyl ring 1 to 5 substituents which may be the same or different and are selected from the group consisting of halogen atoms, $C_{1-6}$alkyl groups and halo-$C_{1-6}$alkyl groups, unsubstituted pyridyloxy group, substituted pyridyloxy groups having 1 to 4 substituents which may be the same or different and are selected from the group consisting of halogen atoms, $C_{1-6}$alkyl groups and halo-$C_{1-6}$alkyl groups, methylenedioxy group, and $C_{3-4}$alkylene groups which each form a bicyclic ring together with the adjacent carbon atoms of the phenyl ring; an unsubstituted benzyloxy group; a substituted benzyloxy group having on the phenyl ring 1 to 5 substituents which may be the same or different and are selected from the group consisting of halogen atoms, $C_{1-6}$alkyl groups, halo-$C_{1-6}$alkyl groups, $C_{1-6}$alkoxy groups, halo-$C_{1-6}$alkoxy groups, $C_{1-6}$alkylthio groups and halo-$C_{1-6}$alkylthio groups; or a 5- or 6-membered heterocyclic ring having 1 to 3 heteroatoms which may be the same or different and are selected from the group consisting of oxygen atom, sulfur atom and nitrogen atom, said heterocyclic ring being able to have one or more substituents selected from the group consisting of $C_{1-6}$alkyl groups, halo-$C_{1-6}$alkyl groups, $C_{1-6}$alkoxy groups and halo-$C_{1-6}$alkoxy groups); unsubstituted amino groups; mono- or di-substituted amino groups having one or two substituents which may be the same or different and are selected from the group consisting of $C_{1-6}$alkyl groups, halo-$C_{1-6}$alkyl groups, $C_{2-6}$alkenyl groups, $C_{2-6}$alkynyl groups, $C_{1-6}$alkoxy-$C_{1-6}$alkyl groups, di-$C_{1-6}$alkoxy-$C_{1-6}$alkyl groups, amino-$C_{1-6}$alkyl groups, substituted mono- or di-$C_{1-6}$alkylamino-$C_{1-6}$alkyl groups having as the substituent(s) one or more $C_{1-6}$alkyl groups which may be the same or different, unsubstituted phenyl group, substituted phenyl groups having 1 to 5 substituents which may be the same or different and are selected from the group consisting of halogen atoms, $C_{1-6}$alkyl groups, $C_{1-6}$alkoxy groups, $C_{1-6}$alkylthio groups and halo-$C_{1-6}$alkyl groups, pyridyl-$C_{1-6}$alkyl groups, and morpholino-$C_{1-6}$alkyl groups; unsubstituted phenyl-$C_{1-6}$alkyl groups; substituted phenyl-$C_{1-6}$alkyl groups having on the phenyl ring 1 to 5 substituents which may be the same or different and are selected from the group consisting of halogen atoms, $C_{1-6}$alkyl groups, halo-$C_{1-6}$alkyl groups, $C_{1-6}$alkoxy groups, halo-$C_{1-6}$alkoxy groups, $C_{1-6}$alkylthio groups and halo-$C_{1-6}$alkylthio groups; unsubstituted phenyl-$C_{2-6}$alkenyl groups; substituted phenyl-$C_{2-6}$alkenyl groups having on the phenyl ring 1 to 5 substituents which may be the same or different and are selected from the group consisting of halogen atoms, $C_{1-6}$alkyl groups and halo-$C_{1-6}$alkyl groups; naphthyl groups; 5- to 7-membered heterocyclic rings having 1 to 3 heteroatoms which may be the same or different and are selected from the group consisting of oxygen atom, sulfur atom and nitrogen atom; or 5- to 7-membered heterocyclic ring fused with a benzene ring or a $C_{4-6}$cycloalkane ring; the above heterocyclic rings being able to have one or more substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxycarbonyl groups, unsubstituted phenyl group, substituted phenyl groups having 1 to 5 substituents which may be the same or different and are selected from halogen atoms, benzyl group, pyridyl group, pyrimidyl group and dioxolane group, $R^3$ and $R^4$ being able to be taken together to represent a $C_{1-6}$-alkylene group or a $C_{2-6}$-alkenylene group, which may contain between adjacent carbon atoms of the carbon chain an oxygen atom, a sulfur atom, a nitrogen atom, a carbonyl group, or a nitrogen atom having a $C_{1-6}$alkyl or phenyl group bonded thereto, said alkylene or alkenylene group being able to have on the carbon chain one or more substituents which may be the same or different and are selected from the group consisting of $C_{1-6}$alkyl groups, halo-$C_{1-6}$alkyl groups, $C_{1-6}$alkoxy groups and halo-$C_{1-6}$alkoxy groups, and to form fused rings with a benzene ring] which comprises reacting a compound represented by the general formula (II):

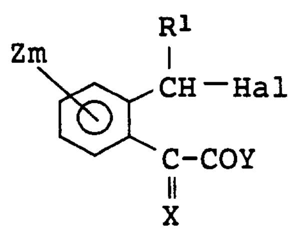

(II)

(wherein X, Y, Z, m and $R^1$ are as defined above, and Hal is a halogen atom) with a compound represented by the general formula (III):

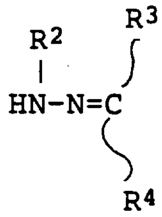

(III)

(wherein $R^2$, $R^3$ and $R^4$ are as defined above).

**6.** A process for producing a benzylhydrazone derivative represented by the general formula (I"):

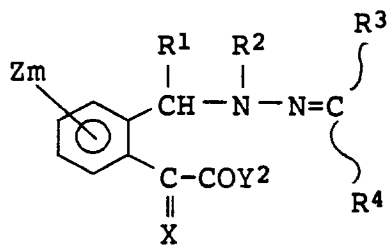

(I")

[wherein X is a group represented by the formula:

CH~$OR^5$

(wherein $R^5$ is a $C_{1-6}$alkyl group) or a group represented by the formula:

N~$OR^5$

(wherein $R^5$ is as defined above), $Y^2$ is an unsubstituted amino group or a mono- or di-substituted amino group having as the substituent(s) one or two $C_{1-6}$alkyl groups which may be the same or different, Z's, which may be the same or different, are halogen atoms, $C_{1-6}$alkyl groups or halo-$C_{1-6}$alkyl groups, m is zero or an integer of 1 to 4, $R^1$ is a hydrogen atom, a $C_{1-6}$alkyl group or a halo-$C_{1-6}$alkyl group, $R^2$ is a formyl group; a $C_{1-6}$alkylcarbonyl group; a halo-$C_{1-6}$alkylcarbonyl group; a $C_{1-6}$alkylsulfonyl group; a halo-$C_{1-6}$alkylsulfonyl group; an unsubstituted aminocarbonyl group; a mono- or di-substituted aminocarbonyl group having one or two substituents which may be the same or different and are selected from the group consisting of formyl group and $C_{1-6}$alkyl groups; a $C_{1-6}$alkoxycarbonyl group; a $C_{1-6}$alkoxycarbonyl-$C_{1-6}$alkylcarbonyl group; a cyano-$C_{1-6}$alkylcarbonyl group; a $C_{1-6}$alkylthiocarbonyl group; an unsubstituted phenylcarbonyl group; a substituted phenylcarbonyl group having 1 to 5 substituents which may be the same or different and are selected from the group consisting of halogen atoms, $C_{1-6}$alkyl groups, halo-$C_{1-6}$alkyl groups, $C_{1-6}$alkoxy groups and halo-$C_{1-6}$alkoxy groups; an unsubstituted phenyl-$C_{1-6}$alkylcarbonyl group; a substituted phenyl-$C_{1-6}$alkylcarbonyl group having on the phenyl ring 1 to 5 substituents which may be the same or different and are selected from the group consisting of halogen atoms, $C_{1-6}$alkyl groups, halo-$C_{1-6}$alkyl groups, $C_{1-6}$alkoxy groups and halo-$C_{1-6}$alkoxy groups; an unsubstituted phenoxycarbonyl group; a substituted phenoxycarbonyl group having on the phenyl ring 1 to 5 substituents which may be the same or different and are selected from the group consisting of halogen atoms, $C_{1-6}$alkyl groups, halo-$C_{1-6}$alkyl groups, $C_{1-6}$alkoxy groups and halo-$C_{1-6}$alkoxy groups; an unsubstituted benzyloxycarbonyl group; a substituted benzyloxycarbonyl group having on the phenyl ring 1 to 5 substituents which may be the same or different and are selected from the group consisting of halogen atoms, $C_{1-6}$alkyl

groups, halo-$C_{1-6}$alkyl groups, $C_{1-6}$alkoxy groups and halo-$C_{1-6}$alkoxy groups; or a di-substituted phosphonyl group having as the substituents two $C_{1-6}$alkyl groups which may be the same or different, and $R^3$ and $R^4$, which may be the same or different, are hydrogen atoms; cyano groups; $C_{1-6}$alkyl groups; halo-$C_{1-6}$alkyl groups; $C_{3-6}$cycloalkyl groups; cyano-$C_{1-6}$alkyl groups; $C_{2-6}$alkenyl groups; $C_{3-6}$cycloalkenyl groups; $C_{1-6}$alkoxy groups; halo-$C_{1-6}$alkoxy groups; $C_{1-6}$alkylthio groups; halo-$C_{1-6}$alkylthio groups; $C_{1-6}$alkylsulfinyl groups; $C_{1-6}$alkylsulfonyl groups; $C_{2-6}$alkenylthio groups; $C_{2-6}$alkynylthio groups; $C_{1-6}$alkoxy-$C_{1-6}$alkyl groups; $C_{1-6}$alkoxy-$C_{1-6}$alkylthio groups; $C_{1-6}$alkylthio-$C_{1-6}$alkyl groups; $C_{1-6}$alkylsulfonyl-$C_{1-6}$alkyl groups; $C_{3-6}$cycloalkylthio groups; $C_{1-6}$alkoxycarbonyl-$C_{1-6}$alkylthio-$C_{1-6}$alkyl groups; $C_{1-6}$alkoxycarbonylthio groups; unsubstituted phenyl groups; substituted phenyl groups having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkylsulfinyl groups, $C_{1-6}$alkylsulfonyl groups, unsubstituted aminocarbonyl group, mono- or di-substituted aminocarbonyl groups having one or two substituents which may be the same or different and are selected from the group consisting of $C_{1-6}$alkyl groups and $C_{1-6}$alkylcarbonyl groups, cyano-$C_{1-6}$alkoxy groups, $C_{1-6}$alkoxycarbonyloxy groups, unsubstituted amino group, mono- or di-substituted amino groups having one or two substituents which may be the same or different and are selected from the group consisting of formyl group, $C_{1-6}$alkyl groups and $C_{1-6}$alkylcarbonyl groups, $C_{1-6}$alkoxycarbonyl groups, unsubstituted aminocarbonyloxy group, mono- or di-substituted aminocarbonyloxy groups having one or two substituents which may be the same or different and are selected from $C_{1-6}$alkyl groups, methylenedioxy group, unsubstituted phenyl group, substituted phenyl groups having 1 to 5 substituents which may be the same or different and are selected from the group consisting of halogen atoms, $C_{1-6}$alkyl groups and $C_{1-6}$alkoxy groups, unsubstituted phenoxy group, substituted phenoxy groups having 1 to 5 substituents which may be the same or different and are selected from the group consisting of halogen atoms, cyano group, nitro group, $C_{1-6}$alkyl groups and halo-$C_{1-6}$alkyl groups, unsubstituted phenyl-$C_{1-6}$alkyl groups, substituted phenyl-$C_{1-6}$alkyl groups having on the phenyl ring 1 to 5 substituents which may be the same or different and are selected from the group consisting of halogen atoms, cyano group, nitro group, $C_{1-6}$alkyl groups and halo-$C_{1-6}$alkyl groups, unsubstituted phenyl-$C_{1-6}$alkoxy groups, substituted phenyl-$C_{1-6}$alkoxy groups having on the phenyl ring 1 to 5 substituents which may be the same or different and are selected from the group consisting of halogen atoms, cyano group, nitro group, $C_{1-6}$alkyl groups and halo-$C_{1-6}$alkyl groups, unsubstituted morpholino group, substituted morpholino groups having as the substituent(s) one or more $C_{1-6}$alkyl groups which may be the same or different, unsubstituted pyrimidinyloxy group, substituted pyrimidinyloxy groups having as the substituent(s) one or more $C_{1-6}$alkyl groups which may be the same or different, unsubstituted morpholinocarbonyl group, substituted morpholinocarbonyl groups having as the substituent(s) one or more $C_{1-6}$alkyl groups which may be the same or different, and $C_{3-4}$alkylene groups which each form a bicyclic ring together with the adjacent carbon atoms of the phenyl ring; unsubstituted phenyl-$C_{1-6}$alkylthio groups; substituted phenyl-$C_{1-6}$alkylthio groups having on the phenyl ring 1 to 5 substituents which may be the same or different and are selected from the group consisting of $C_{1-6}$alkyl groups and halo-$C_{1-6}$alkyl groups; unsubstituted phenylsulfonyl groups; substituted phenylsulfonyl groups having on the phenyl ring 1 to 5 substituents which may be the same or different and are selected from the group consisting of $C_{1-6}$alkyl groups and halo-$C_{1-6}$alkyl groups; unsubstituted phenylsulfonyl-$C_{1-6}$alkyl groups; substituted phenylsulfonyl-$C_{1-6}$alkyl groups having on the phenyl ring 1 to 5 substituents which may be the same or different and are selected from the group consisting of $C_{1-6}$alkyl groups and halo-$C_{1-6}$alkyl groups; unsubstituted phenylthio-$C_{1-6}$alkyl groups; substituted phenylthio-$C_{1-6}$alkyl groups having on the phenyl ring 1 to 5 substituents which may be the same or different and are selected from the group consisting of $C_{1-6}$alkyl groups and halo-$C_{1-6}$alkyl groups; unsubstituted phenyl-$C_{1-6}$alkylthio-$C_{1-6}$alkyl groups; substituted phenyl-$C_{1-6}$alkylthio-$C_{1-6}$alkyl groups having on the phenyl ring 1 to 5 substituents which may be the same or different and are selected from the group consisting of $C_{1-6}$alkyl groups and halo-$C_{1-6}$alkyl groups; unsubstituted amino-$C_{1-6}$alkyl groups; mono- or di-substituted amino-$C_{1-6}$alkyl groups having one or two substituents which may be the same or different and are selected from the group consisting of $C_{1-6}$alkyl groups, halo-$C_{1-6}$alkyl groups, $C_{1-6}$alkylcarbonyl groups, unsubstituted phenylcarbonyl group, substituted phenylcarbonyl groups having on the phenyl ring 1 to 5 substituents which may be the same or different and are selected from the group consisting of $C_{1-6}$alkyl groups and halo-$C_{1-6}$alkyl groups, phenyl group and benzyl group; furfurylthio groups; furfurylthio-$C_{1-6}$alkyl groups; unsubstituted morpholino-$C_{1-6}$alkyl groups; substituted morpholino-$C_{1-6}$alkyl groups having as the substituent(s) one or more $C_{1-6}$alkyl groups which may be the same or different; groups represented by the formula:

-CO-$R^6$

(wherein R6 is a $C_{1-6}$alkyl group; a halo-$C_{1-6}$alkyl group; a $C_{1-6}$alkoxy group; a halo-$C_{1-6}$alkoxy group; a $C_{1-6}$alkylthio group; a halo-$C_{1-6}$alkylthio group; an unsubstituted amino group; a mono- or di-substituted amino group having one or two substituents which may be the same or different and are selected from the group consisting of $C_{1-6}$alkyl groups, halo-$C_{1-6}$alkyl groups and phenyl group; an unsubstituted phenyl group; a substituted phenyl group having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, unsubstituted amino group, mono- or di-substituted amino groups having one or two substituents which may be the same or different and are selected from the group consisting of $C_{1-6}$alkyl groups and $C_{1-6}$alkylcarbonyl groups, unsubstituted phenyl group, substituted phenyl groups having 1 to 5 substituents which may be the same or different and are selected from the group consisting of halogen atoms, $C_{1-6}$alkyl groups and halo-$C_{1-6}$alkyl groups, unsubstituted phenoxy group, substituted phenoxy groups having 1 to 5 substituents which may be the same or different and are selected from the group consisting of halogen atoms, cyano group, nitro group, $C_{1-6}$alkyl groups and halo-$C_{1-6}$alkyl groups, benzyl group, unsubstituted benzyloxy group, substituted benzyloxy groups having on the phenyl ring 1 to 5 substituents which may be the same or different and are selected from the group consisting of halogen atoms, $C_{1-6}$alkyl groups and halo-$C_{1-6}$alkyl groups, unsubstituted pyridyloxy group, substituted pyridyloxy groups having 1 to 4 substituents which may be the same or different and are selected from the group consisting of halogen atoms, $C_{1-6}$alkyl groups and halo-$C_{1-6}$alkyl groups, methylenedioxy group, and $C_{3-4}$alkylene groups which each form a bicyclic ring together with the adjacent carbon atoms of the phenyl ring; an unsubstituted benzyloxy group; a substituted benzyloxy group having on the phenyl ring 1 to 5 substituents which may be the same or different and are selected from the group consisting of halogen atoms, $C_{1-6}$alkyl groups, halo-$C_{1-6}$alkyl groups, $C_{1-6}$alkoxy groups, halo-$C_{1-6}$alkoxy groups, $C_{1-6}$alkylthio groups and halo-$C_{1-6}$alkylthio groups; or a 5- or 6-membered heterocyclic ring having 1 to 3 heteroatoms which may be the same or different and are selected from the group consisting of oxygen atom, sulfur atom and nitrogen atom, said heterocyclic ring being able to have one or more substituents selected from the group consisting of $C_{1-6}$alkyl groups, halo-$C_{1-6}$alkyl groups, $C_{1-6}$alkoxy groups and halo-$C_{1-6}$alkoxy groups); unsubstituted amino groups; mono- or di-substituted amino groups having one or two substituents which may be the same or different and are selected from the group consisting of $C_{1-6}$alkyl groups, halo-$C_{1-6}$alkyl groups, $C_{2-6}$alkenyl groups, $C_{2-6}$alkynyl groups, $C_{1-6}$alkoxy-$C_{1-6}$alkyl groups, di-$C_{1-6}$alkoxy-$C_{1-6}$alkyl groups, amino-$C_{1-6}$alkyl groups, substituted mono- or di-$C_{1-6}$alkylamino-$C_{1-6}$alkyl groups having as the substituent(s) one or more $C_{1-6}$alkyl groups which may be the same or different, unsubstituted phenyl group, substituted phenyl groups having 1 to 5 substituents which may be the same or different and are selected from the group consisting of halogen atoms, $C_{1-6}$alkyl groups, $C_{1-6}$alkoxy groups, $C_{1-6}$alkylthio groups and halo-$C_{1-6}$alkyl groups, pyridyl-$C_{1-6}$alkyl groups, and morpholino-$C_{1-6}$alkyl groups; unsubstituted phenyl-$C_{1-6}$alkyl groups; substituted phenyl-$C_{1-6}$alkyl groups having on the phenyl ring 1 to 5 substituents which may be the same or different and are selected from the group consisting of halogen atoms, $C_{1-6}$alkyl groups, halo-$C_{1-6}$alkyl groups, $C_{1-6}$alkoxy groups, halo-$C_{1-6}$alkoxy groups, $C_{1-6}$alkylthio groups and halo-$C_{1-6}$alkylthio groups; unsubstituted phenyl-$C_{2-6}$alkenyl groups; substituted phenyl-$C_{2-6}$alkenyl groups having on the phenyl ring 1 to 5 substituents which may be the same or different and are selected from the group consisting of halogen atoms, $C_{1-6}$alkyl groups and halo-$C_{1-6}$alkyl groups; naphthyl groups; 5- to 7-membered heterocyclic rings having 1 to 3 heteroatoms which may be the same or different and are selected from the group consisting of oxygen atom, sulfur atom and nitrogen atom; or 5- to 7-membered heterocyclic ring fused with a benzene ring or a $C_{4-6}$cycloalkane ring; the above heterocyclic rings being able to have one or more substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxycarbonylgroups, unsubstituted phenyl group, substituted phenyl groups having 1 to 5 substituents which may be the same or different and are selected from halogen atoms, benzyl group, pyridyl group, pyrimidyl group and dioxolane group, $R^3$ and $R^4$ being able to be taken together to represent a $C_{1-6}$-alkylene group or a $C_{2-6}$-alkenylene group, which may contain between adjacent carbon atoms of the carbon chain an oxygen atom, a sulfur atom, a nitrogen atom, a carbonyl group, or a nitrogen atom having a $C_{1-6}$alkyl or phenyl group bonded thereto, said alkylene or alkenylene group being able to

have on the carbon chain one or more substituents which may be the same or different and are selected from the group consisting of $C_{1-6}$alkyl groups, halo-$C_{1-6}$alkyl groups, $C_{1-6}$alkoxy groups and halo-$C_{1-6}$alkoxy groups, and to form fused rings with a benzene ring] which comprises reacting a benzylhydrazone derivative represented by the general formula (I'):

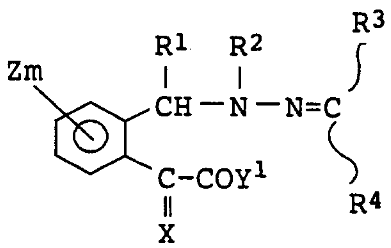

(I')

(wherein X, Z, m, $R^1$, $R^2$, $R^3$ and $R^4$ are as defined above, and $Y^1$ is a $C_{1-6}$alkoxy group or a $C_{1-6}$alkylthiogroup) with a compound represented by the general formula (IV):

$Y^2$-H

(wherein Y2 is as defined above).

**7.** An agricultural and horticultural fungicide comprising a benzylhydrazone derivative set forth in claim 1 as an active ingredient.

**8.** An agricultural and horticultural fungicide comprising a benzylhydrazone derivative set forth in claim 2 as an active ingredient.

**9.** An agricultural and horticultural fungicide comprising a benzylhydrazone derivative set forth in claim 3 as an active ingredient.

**10.** An agricultural and horticultural fungicide comprising a benzylhydrazone derivative set forth in claim 4 as an active ingredient.